(19) Europäisches Patentamt
European Patent Office
Office européen des brevets

(11) EP 3 490 673 B1

(12) **EUROPEAN PATENT SPECIFICATION**

(45) Date of publication and mention
of the grant of the patent:
**22.01.2025 Bulletin 2025/04**

(21) Application number: **17835325.6**

(22) Date of filing: **28.07.2017**

(51) International Patent Classification (IPC):
**A61N 7/00** (2006.01)  **A61K 35/12** (2015.01)

(52) Cooperative Patent Classification (CPC):
**A61N 7/00; A61K 35/19; A61K 35/50;
A61M 37/0092; A61P 17/02;** A61B 90/96;
A61B 90/98; A61B 2090/0803; A61B 2090/0814;
A61B 2218/002; A61F 2007/0059;
A61M 2037/0007; A61N 2007/0017

(86) International application number:
**PCT/US2017/044323**

(87) International publication number:
**WO 2018/022968 (01.02.2018 Gazette 2018/05)**

(54) **SYSTEMS FOR DELIVERING CELLULAR AND BIOLOGICAL MATERIALS USING ULTRASOUND FOR WOUND TREATMENT AND HEALING**

SYSTEME ZUR BEREITSTELLUNG VON ZELL- UND BIOLOGISCHEN MATERIALIEN MITTELS ULTRASCHALL ZUR WUNDBEHANDLUNG UND WUNDHEILUNG

SYSTÈMES POUR ADMINISTRER DES MATÉRIAUX CELLULAIRES ET BIOLOGIQUES AU MOYEN D'ULTRASONS POUR LE TRAITEMENT ET LA CICATRISATION DE PLAIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2016 US 201662368297 P**

(43) Date of publication of application:
**05.06.2019 Bulletin 2019/23**

(73) Proprietor: **Alliqua Biomedical, Inc.**
Yardley, Pennsylvania 19067 (US)

(72) Inventors:
• DUCHON, Douglas J.
Yardley
Pennsylvania 19067 (US)
• BHATIA, Mohit
Yardley
Pennsylvania 19067 (US)
• SMIELL, Janice M.
Yardley
Pennsylvania 19067 (US)
• TETZLOFF, Ryan Glen
Yardley
Pennsylvania 19067 (US)

(74) Representative: **D Young & Co LLP**
3 Noble Street
London EC2V 7BQ (GB)

(56) References cited:
EP-A1- 4 095 228         WO-A1-2016/033041
WO-A1-2016/033041       WO-A1-2016/065341
WO-A2-2009/005980       US-A1- 2004 259 053
US-A1- 2006 241 533      US-A1- 2007 073 197
US-A1- 2009 043 248      US-A1- 2009 043 248
US-A1- 2012 010 506      US-A1- 2012 046 601
US-A1- 2014 335 046      US-A1- 2015 148 712
US-A1- 2015 148 712      US-B2- 6 623 444
US-B2- 6 960 173         US-B2- 8 647 720
US-B2- 9 365 341

• ANONYMOUS: "Quick Reference Guide",
INTERNET CITATION, 31 May 2015 (2015-05-31),
pages 1, XP009518740, Retrieved from the
Internet <URL:https://web.archive.
org/web/20160109134609/http://amemark.com/>

EP 3 490 673 B1

• **ANONYMOUS: "Quick Reference Guide", June 2015 (2015-06-01), UNITED STATES, XP009518740, Retrieved from the Internet <URL:https://web.archive. org/web/20160109134609/http://amemark.com/>**

## EP 3 490 673 B1

**Description**

TECHNICAL FIELD

Embodiments relate generally to systems for delivering cellular and/or biological materials using ultrasound for wound treatment and healing, and more particularly to a non-contact, low-frequency, highly efficient ultrasound therapy and biologic delivery system that delivers ultrasonic therapy treatments while distributing and biologic or cellular materials via a mist to a patient skin or tissue to promote healing and provide a cover to denuded, damaged and/or inadequate tissues.

BACKGROUND

Use of ultrasonic energy in the treatment of wounds has become more common in recent years as its benefits are better understood and this type of therapy becomes more widely utilized. In general, ultrasonic waves of various frequencies and intensities have been used in medical applications for a long time, including diagnostics, therapy, and industrial applications.

A number of innovative ultrasound therapy systems and devices have previously been developed. These systems and devices have been widely used for medical treatments in medical facilities around the world. See, for example, U.S. Patent No. 6,569,099, entitled ULTRASONIC METHOD AND DEVICE FOR WOUND TREATMENT. Unlike most conventional wound therapies that are limited to treatment of the wound surface, this patent discusses therapies in which ultrasound energy and atomized normal saline solutions were used to stimulate the cells within and below the wound bed to aid in initiating or promoting the healing process.

Although these ultrasound therapies have been effective, devices, systems and methods providing improved ultrasonic therapies that are more accessible, safer and easier to administer to patients, and more efficient in delivery of ultrasound energy have been desired.

Biologic products, including extracellular matrices, naturally occurring proteins, growth factors and adherent cell populations, are important components in the healing process. Additionally, further administration of biologic materials, either taken from autologous or allogenic sources, are capable of covering, supporting and/or enhancing the healing progress of partial- or full-thickness wounds. Moreover, some biologic materials are capable of being administered topically. Administration of some biologic components may result in modulation of inflammation that is additive or synergistic with that of ultrasound energy.

However, the effectiveness of biologic treatment of wounds is often limited because many of the healing components are unable to penetrate the epidermal, dermal or subcutaneous tissues. Most topically applied agents or cellular materials cannot normally penetrate the skin (intact or disrupted) with current methods and cannot be transported into the dermis. US2015/148712, US2007/073197, US2014/335046, US2009/043248 and US2012/046601 refer to prior art relevant for the present invention.

SUMMARY

The present invention is defined by appended claim 1. Preferred embodiments are disclosed in the dependent claims.

Embodiments relate to biologic and/or cellular material delivery and non-contact, low-frequency, highly efficient ultrasound therapy devices, and systems that deliver ultrasonic and biologic therapy treatments via a mist to a patient wound to promote wound healing. One embodiment is directed to a non-contact, medical ultrasound therapy system for generating and controlling low frequency ultrasound alongside delivery of biologic for promoting wound healing. The ultrasound therapy system includes a treatment wand including an ultrasonic transducer, a generator unit, a cable coupling the treatment wand to the generator unit, and a biologic and cellular material delivery mechanism. The generator unit generates electric power output to drive the ultrasonic transducer and includes a digital frequency generator, wherein the generator unit digitally controls energy output at resonance frequency of the ultrasonic transducer.

The disclosure also provides methods, presently not claimed, for treating a skin, mucosa, or other condition. In some embodiments, these methods involve using an ultrasound delivery device to apply to an area of skin, mucosa or other tissue affected by the condition a mist that comprises a micronized cellular or biological material. The skin or other condition can be, for example, a wound, ulcer, or other condition. The cellular or biological material is, in some embodiments, a placental extracellular matrix composition or a placental connective tissue matrix composition. These compositions can be prepared from, for example, whole placental, placental deciduas, placental amniotic membrane, or placental chorionic membrane. In some embodiments, the biological material includes a population of adherent cells, or a mixture of adherent and non-adherent cells. The cellular or biological material can also include platelet-rich plasma or placental perfusate. The cellular or biological material is micronized in some embodiments of the invention.

According to the invention, medical ultrasound devices are provided for delivering non-contact ultrasound therapies to a skin or other condition. These devices include at least one treatment wand that includes an ultrasonic transducer, at least one reservoir that contains a fluid or suspension that includes a micronized cellular or biological material; and a pump that is in fluid communication with the reservoir and the treatment wand to deliver the fluid to the treatment wand such that the ultrasonic transducer atomizes the cellular or biological material as the cellular or biological material passes through the treatment wand for delivery to the area of skin or other tissue that is affected by the condition. In some embodiments, the devices of the invention have a plurality of treatment wants and/or a plurality of reservoirs. Each treatment wand can be in fluid communication with one reservoir, or a treatment wand can be in fluid communication with more than one reservoir. In some embodiments, at least one of the reservoirs contains a fluid for cleaning or disinfecting a wound or other tissue. A fluid for debriding a wound or tissue, or a fluid for providing a protective or other coating on a wound or other tissue, can also be contained within one or more of the reservoirs. The reservoir(s) are sterile, in some embodiments, and the fluid that is contained in the reservoir(s) can also be sterile.

The medical ultrasound devices of the invention, in some embodiments, further include an applicator configured to be coupled to the treatment wand, wherein the applicator has a radio frequency identification (RFID) tag and the treatment wand has a RFID transceiver that is used to identify the RFID tag on the applicator to ensure that the applicator is limited to a single use.

The devices of the invention also include, in some embodiments, a microprocessor that is configured to control operation of the device. The treatment wand can include, in some embodiments, a plurality of tubes and a plurality of reservoirs, each tube in fluid communication with a different one of the plurality of reservoirs, and a microprocessor that is configured to control a delivery pattern of fluids from the plurality of reservoirs. For example, the delivery pattern can be, in some embodiments, sequential delivery of individual fluids or simultaneous delivery of at least two fluids. The devices can also include at least one valve that is configured to selectively couple at least one of the reservoirs to a treatment wand. The valve can include, for example, a static mixer. The microprocessor can be configured to control operation of the valve(s) and the device. The valve can also be manually controllable.

BRIEF DESCRIPTION OF THE DRAWINGS

The disclosure may be more completely understood in consideration of the following detailed description of various embodiments of the invention in connection with the accompanying drawings, in which:

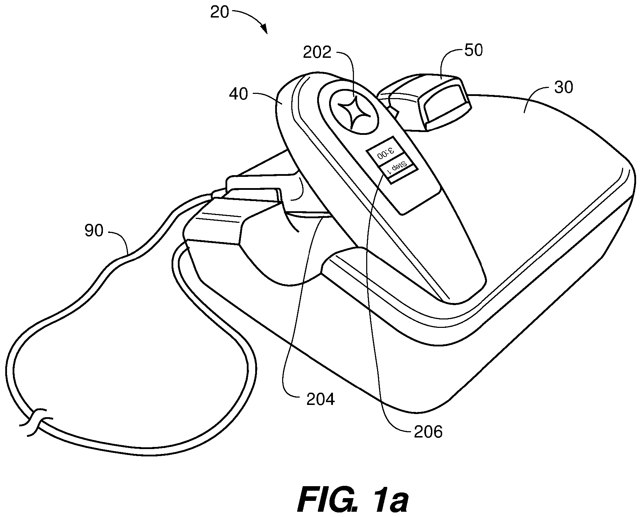

FIG. 1a is an ultrasound device of a system providing non-contact therapy to patient wounds via a low frequency ultrasound mist, according to an embodiment.

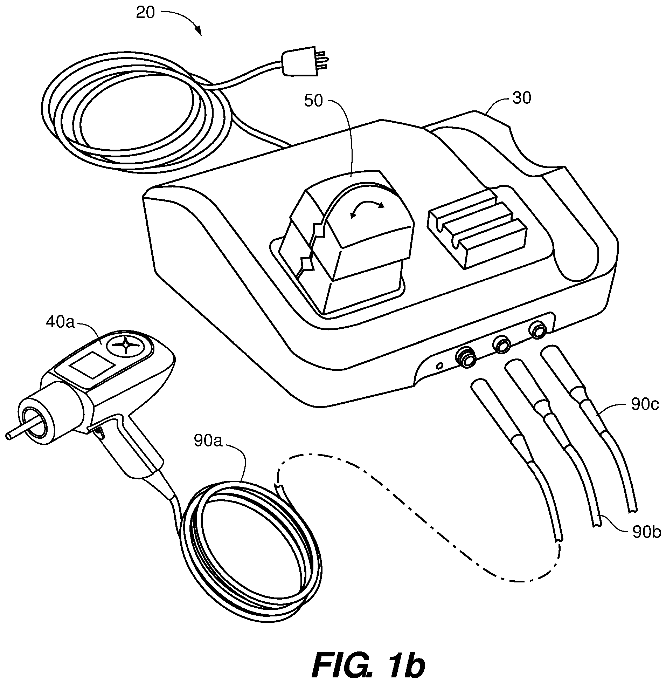

FIG. 1b is another ultrasound device of a system providing non-contact therapy to patient wounds via a low frequency ultrasound mist, according to an embodiment.

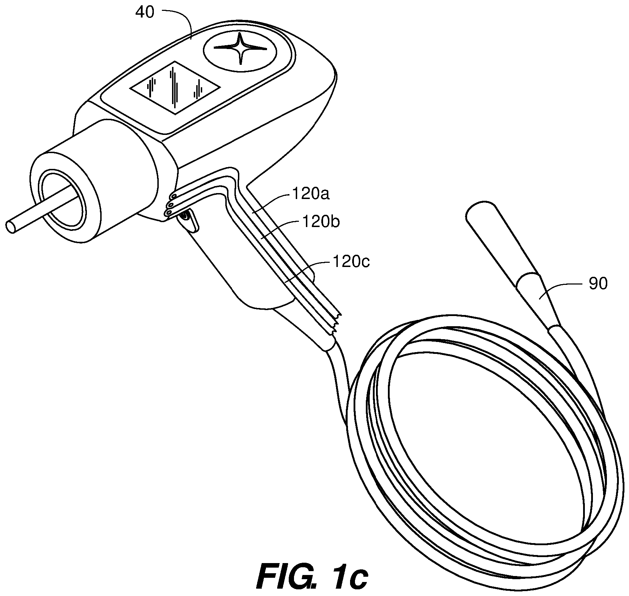

FIG. 1c is a treatment wand for use in a system providing non-contact therapy to patient wounds via a low frequency ultrasound mist, according to an embodiment.
FIGS. 1d-1 - 1d-5 are a valve device for use in a system providing non-contact therapy to patient wounds via a low frequency ultrasound mist, according to an embodiment.
FIGS. 1e-1 - 1e-4 are a nozzle/applicator for use in a system providing non-contact therapy to patient wounds via a low frequency ultrasound mist, according to an embodiment.
FIGS. 1f-1 - 1f-4 are a pump for use in a system providing non-contact therapy to patient wounds via a low frequency ultrasound mist, according to an embodiment.
FIG. 1g is a temperature management device for use in a system providing non-contact therapy to patient wounds via a low frequency ultrasound mist, according to an embodiment.
FIG. 2 is a diagram of an ultrasound therapy system, according to an embodiment.
FIG. 3 is a diagram of an ultrasound therapy system, according to an embodiment.
FIG. 4 is a diagram of an ultrasound device of a system providing for non-contact therapy to patient wounds via a low frequency ultrasound mist,cording to an embodiment.
FIG. 5 is a diagram of the interaction of the DDS (Direct Digital Synthesis) feature and microprocessor that provides digital frequency generation, according to an embodiment.
FIG. 6 is a diagram of the frequency control loop of the system, according to an embodiment.
FIG. 7 is a diagram of the constant current control loop of the system, according to an embodiment.
FIG. 8 is a graph of an example of impedance versus frequency, in an ultrasonic transducer device, according to an embodiment.
FIGS. 9a - 9g show diagrams of the operation of the ultrasonic therapy system, according to an embodiment.
FIGS 10a - 10d show diagrams of application and treatment of the biologic/cellular material, according to some embodiments.

DETAILED DESCRIPTION

Various embodiments may be embodied in other specific forms without departing from the essential attributes thereof, therefore, the illustrated embodiments should be considered in all respects as illustrative and not restrictive.

Skin and/or mucosal ulcers or wounds, which are breaks in the skin/mucosa located anywhere on the surface of the body or its cavities, are a common occurrence. Healing of these wounds may be complicated by concomitant disease states, making them difficult or slow to heal (i.e., chronic). Wounds may be acute but difficult to treat because of location or association with concomitant conditions or injuries and require more innovative treatment than simple or routine good wound care to accomplish a functional regenerative repair or significant pain relief. Acute wounds are usually the result of accidental (e.g., burn or trauma) or iatrogenic (e.g., surgery) injuries. Chronic wounds afflict certain patient populations, particularly the elderly, immobile, obese, under-exercised, or with comorbidities like diabetes, atherosclerosis or autoimmune/inflammatory conditions, while acute wounds are ubiquitous. Chronic wounds may initiate as acute wounds that are complicated by the presence of a disease state or become infected, causing them to require more than four to six weeks to heal. Chronic wounds are persistent, non-healing or slow-healing wounds in which the body's healing process becomes stalled. They require treatment of the underlying disease state in parallel to treatment of the wound to achieve healing. In order to maximize the potential for a functional outcome, a regenerative healing process is desired. To reduce scarring or fibrotic repair of wound, application of human tissue components that maintain their architecture and/or biochemical construct have been shown to increase the likelihood of a regenerative repair that minimizes fibrosis and maximizes the functional and aesthetic outcomes.

Skin ulcers, particularly leg ulcers (especially venous stasis and diabetic foot ulcers), have been treated by application of a variety of skin substitutes or dressings, such as xenografts, which can include extracellular matrix products derived from porcine/bovine/equine/ovine/piscine sources. Conventional products include living bi-layered skin substitutes comprised of dermal layer and epidermal layers with a synthetic and/or animal-derived matrix, human acellular dermal matrix products that are derived from donated human skin, or donated human skin that is cryopreserved to maintain living cells in the bilayer construct. Dressings that are considered skin substitutes include the products that include plant-sourced advanced matrices. The skin substitutes, however, have disadvantages that include difficulty in handling, storing, sourcing or manufacturing challenges and size limitations. Thus, despite the prevalence of skin wounds, particularly chronic ulcerations, and the availability of different types of graft materials, there exists a need for a method of supporting or promoting the healing of wounds using a flexible, readily-available, customizable, easily applied durable material that can facilitate healing of the ulcerated or wounded area. Other needs also exist, including for treating conditions other than wounds (e.g., psoriasis, eczema, and other rashes and skin conditions that significant inflammation associated therewith). Thus, while wounds may be used as examples herein throughout, treatment areas need not comprise wounds *per se* and can comprise areas of the skin or body afflicted by some condition or otherwise viewed by a medical professional as potentially responsive to therapy or treatment.

These and other needs for more accessible and effective biologic and cellular material delivery and therapy for patients have been recognized in this disclosure. In embodiments, these needs can be substantially addressed or met by a low-frequency ultrasound therapy device and system configured for use with biologic and cellular material delivery and therapy. Many of the substantial technical obstacles to providing such a device based on the requirements of conventional ultrasound therapy devices are recognized and overcome by this disclosure. Specifically, making devices more readily accessible to additional patient populations by treating a more diverse set of ailments or conditions with new materials and techniques has been a significant challenge now met, at least in part, by embodiments discussed in this disclosure.

An additional challenge associated with conventional ultrasound therapy devices is the very high voltage necessary to operate conventional devices. For example, some conventional ultrasound therapy devices have operated at about 700 Volts (V) and 7 Watts (W) of energy. This has necessitated qualified oversight of therapy provision, as allowing untrained users to operate such a high voltage machine on their own might otherwise present a significant safety risk. The energy requirements have made the possibility of a portable battery powered device, which could be used in a homecare or other nonclinical environment, unfeasible.

Further, many patients cannot tolerate conventional topical applications of biologic or cellular materials due to direct physical contact with the wound and surrounding area during the procedure, and/or those conventional applications are less effective than desired. Delivery of biologic or cellular and biologic materials using ultrasound therapy systems described herein, however, address many or all of the technological, patient tolerance, and clinical effectiveness obstacles of the past and provide a lower-power, safer, more efficient, and more accessible ultrasound system for delivery of and therapy by biologic or cellular materials. Even battery powered systems are possible in certain embodiments. In embodiments, treatment, acceleration, and support or promotion of healing may be achieved by providing an optimal selection of ultrasound parameters such as frequency, intensity, pulse length, beam characteristics, and application time on the skin to enhance the transportation of biological cellular agents into the epidermal, dermal and subcutaneous tissues. Thus, topical agents of cellular materials that cannot normally penetrate the skin or mucosa with current methods may be transported into the dermis or submucosa. Accordingly, designs for new biologic and cellular material delivery and medical ultrasound devices, systems and methods incorporating various features, concepts and improvements, are discussed in the following pages.

Cellular and/or biological materials suitable for use in embodiments can be prepared and delivered in many formats. Biologic materials or viable tissue can be harvested and processed via several methodologies in order to create a fine particulate. In some embodiments, micronized or particulate cellular or biological materials are created through an aseptic mechanical milling process that creates a desired or appropriate size of particle. Examples of suitable milling processes include ball milling, jet milling, impact milling and friction milling. Other milling or processing methodologies to micronize the cellular or biological material can be used in other embodiments, such as grinding.

In embodiments, and regardless of the processing methodology used, the particles of cellular or biological material can be sized to be smaller than a delivery orifice of a delivery mechanism of the ultrasound device, or the delivery orifice can be selected for use according to a known particulate size in a specific application. In embodiments, the particle size is less than about 60 micrometers ($\mu$m), for example less than about 30 $\mu$m in some embodiments, or between about 20 $\mu$m and 30$\mu$m in one embodiment. The particle size also can be considered relative to atomized droplet size, when a fluid in which undissolved particles are suspended is atomized. In one embodiment, the particle size is less than or about 1/10 of the size of the atomized droplet. Particle size also can affect or depend upon the viscosity of the fluid in which the particles are suspended or dissolved, and fluids with viscosities of less than or about 100 centipoise can be advantageous in some embodiments, though fluids with higher viscosities still may be used in other embodiments. Because atomized droplet size depends on fluid viscosity and frequency, adjustments can be made to one or more characteristics, for different applications or situations.

In some aspects, various tissues and cells can be combined to create a mixture of biologics that are optimized for a particular application, therapy, patient or other characteristic. Additionally, characteristics of the fluid for atomization and/or the device that atomizes the fluid can be adjusted or optimized for a particular application, therapy, patient or other characteristic.

The cellular or biological material can comprise a population of an adherent cells, such as fibroblasts or mesenchymal stem cells (MSCs). The cells can be a mix of different cell types or a homogenous population. In some embodiments, cells can be derived from the patient or placental sources. Patient-sourced cells can be taken from a biopsy of skin or other tissue, and the tissue can be homogenized. The cells can be separated using a variety of commercial methods including plating cells on a tissue culture plate to enrich an adherent cell population. In other embodiments, the cellular or biological material can comprise a mixture of adherent and non-adherent cells.

There are two typical placental cell sources. First, perfusate sources contain a mix of adherent and non-adherent cell types. The perfusate can be plated on tissue culture plates, populated and later removed using a variety of well-known methods, including the use of a collagenase solution. The cells can be diluted into an appropriate pH balanced buffer, such as phosphate-buffered saline. An additive can be added to the cell buffer to improve cell viability and passage through the ultrasound device. Second, the placenta tissue itself can serve as a source of cells. Cells can be derived from an umbilical cord, the Wharton's jelly or the placental decidua. The tissue can be scraped and partly homogenized using mechanical or chemical methodologies, followed by plating the cells upon a tissue culture plate. The cells can be removed from the plate and mixed with a benign buffer. Cells can be autologous or allogeneic with immunomodulatory properties.

Biologic materials also can be derived from the placenta via the connective tissue matrix or the placental extracellular matrix to form a placental connective tissue matrix composition or a placental extracellular matrix composition. The connective tissue matrix can be derived from part of the placenta and processed within the guidance of the current good tissue practices (CGTPs). Once excised from the placenta, the materials can be ground and washed with salt and a detergent, such as deoxycholic acid, in order to produce a paste. The paste can then be lyophilized and jet-milled to a particle size of below that of the delivery mechanism aperture. Another method of preparing a connective tissue matrix includes preparing a soluble form. The connective tissue matrix can be dissolved using hydrochloric acid in solution around a pH of 2.0. Phosphate-buffered solution can then be applied immediately following application of the connective tissue matrix at a pH about 7.2.

Whole placental extracellular matrix can be generated using the full or whole placenta before or after removal of the amnion and the umbilical cord. The whole placental tissue can be ground and repeatedly washed with salt and water and then with deoxycholic acid. The tissue can be exposed to a low pH (e.g., pH = 3.0) and a high pH (e.g., pH = 13.0). The tissue can be washed with salt and water, forming a paste. The paste can be jet milled or ground. The tissue can be dissolved in hydrochloric acid solution with a pH of about 2.0.

Placental extracellular matrix or placental connective tissue matrix also can be prepared from placental amniotic membrane or placental chorionic membrane.

The cellular or biological materials can be prepared in different forms, such as, for example, being micronized by grinding, milling (e.g., jet milling), freeze drying, or heat drying. The materials also can be solubilized. The cellular or biological material can be solubilized or otherwise made compatible for delivery via an ultrasound system in advance of or at the time of delivery and ultrasound therapy, when the material can be applied as part of a fluid, mist, or topical treatment. In some embodiments, the material application can be followed by administration of a solvent, application of further ultrasound therapy, or other treatments.

For example, in one embodiment a connective tissue matrix or extracellular matrix can be suspended in a solution and jet milled with saline or another fluid to a particle size below that of the aperture of the ultrasound delivery systems. In another embodiment, a biologic material can be applied as a powder to the wound or other area of patient anatomy where it is desired or necessary. Saline solution then can be applied to the powder, followed by ultrasound therapy provided by the ultrasound system to facilitate an even distribution and penetration of the biological material into deeper tissue levels, e.g., the dermis and subdermal levels of skin. In yet another embodiment, a biologic material can be freeze-dried or heat-dried. The dried material then can be applied to a wound or other area of patient anatomy where it is desired or necessary as a sheet or layer, followed by treatment with saline solution or another compatible fluid and ultrasound treatment to facilitate an even distribution and penetration of the biologic material into deeper tissue levels. In still another embodiment, a biologic material can be an acid solubilized solution of connective tissue matrix or extracellular matrix, treated with saline, phosphate buffered saline or another fluid, and delivery using ultrasound therapy.

In some embodiments, the cellular or biological material can comprise a platelet rich plasma or placental perfusate. Platelet rich plasma can be autologous and can be isolated using known methods. In one embodiment, the plasma is applied prior to ultrasound treatment, with or without saline treatment. Placenta perfusate can be generated, for example, by passing normal saline through the placenta via the umbilical cord blood vessels. The perfusate can be collected as a mix of proteins and cells in saline. The proteins can include blood proteins, growth factors and cytokines, although these may not be characterized. The cells can be a mix of CD34 hematopoietic progenitors, stems cells and adherent cell types. The cell composition of the perfusate need not be well characterized. Cell size can be a size capable of passing through the aperture of the delivery mechanism. In use, biological fluids such as platelet-rich blood plasma, autologous blood plasma, and placenta perfusate can be applied via the delivery device with or without saline treatment.

Still other materials and combinations thereof can be used in other embodiments. Thus, for example, materials comprising or containing, together or separated (i.e., purified), alone or combined, any of amnion, chorion, umbilical cord/cord blood derivatives, placental decidua, epithelium, basement membrane, extracellular matrix (ECM), connective tissue matrix (CTM, such as INTERFYL™), scaffold protein, plasma, collagen, elastin, platelet-rich plasma (PRP), glycosaminoglycan (GAG), proteoglycan (PG), laminins, fibronectin, cytokines, hemagglutinin (HA), coated fibers, other molecules with placental-derived components, antibiotics, anti-inflammatories, antifungals, and other cellular or biologic materials or combinations thereof can be used in various embodiments. As appreciated by those having skill in the art, the handling, storage, sterilization and combination, as well as the therapeutic delivery using ultrasound as discussed herein, of these materials can vary.

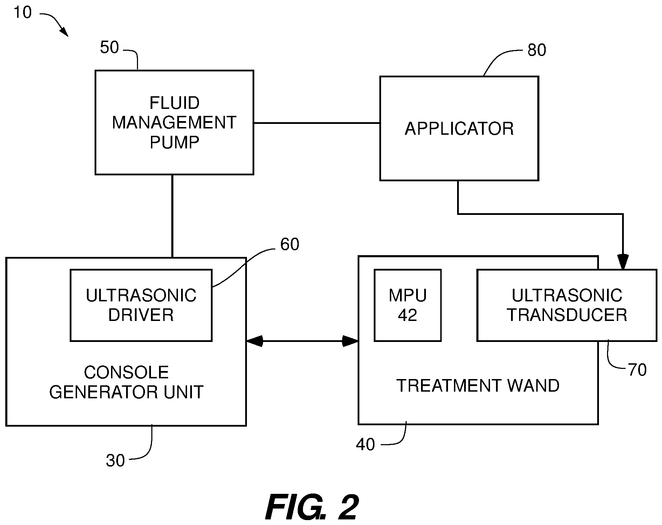

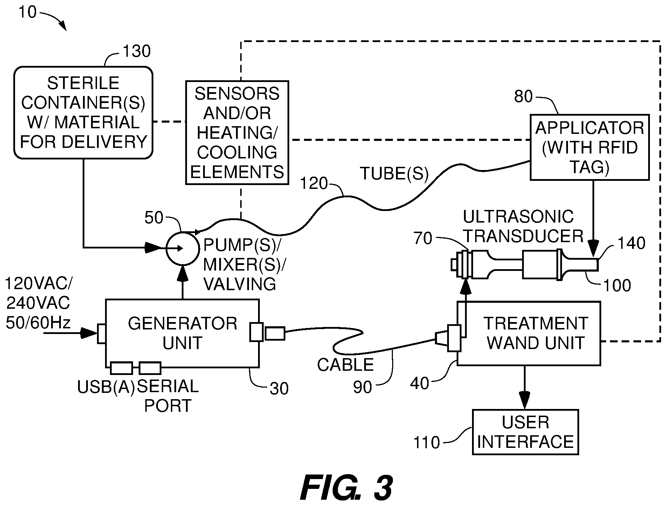

FIG. 1a shows an example of a medical ultrasound device 20 of an ultrasound therapy system 10 (refer, e.g., to FIG. 2) for delivering non-contact ultrasound therapies to patient wounds via a low-frequency ultrasound mist. Medical ultrasound device 20 comprises both a console/generator unit 30 for generating power and a treatment wand 40 for administering therapies. In general, generator unit 30 supplies power to an ultrasonic transducer within the treatment wand 40. Treatment wand 40 is generally and ergonomically pistol-shaped and may be conveniently positioned by a user to direct ultrasonic energy to a treatment area via atomized saline mist emitted from the end of treatment wand 40. Generator unit 30 further comprises one or more external pumps 50 to pump saline, cellular/biologic and/or other materials through a tube or tubes 120 (see, e.g., FIG. 3) attached to the end of treatment wand 40. Pumps 50 depicted in FIG. 1a are peristaltic pumps but can comprise other suitable pump types or mechanism in other embodiments, such as rotary/gear pumps, positive displacement syringe pumps, or other pumps.

In some aspects of the invention, the system can have multiple treatment wands 40 and/or multiple flow channels. These configurations can allow for sequenced or simultaneous delivery of various materials, different treatment modalities or characteristics, expanded treatment areas, and other advantages.

One such embodiment is depicted in FIG. 1b. In the embodiment of FIG. 1b, system 10 can comprise a plurality of treatment wands 40 (one wand 40a is depicted in FIG. 1b), each of which can be coupled to a single generator unit 30 of device 20 by respective cables 90a, 90b and 90c. In use, the plurality of treatment wands 40 can be operated simultaneously or sequentially, and each wand 40 can be used to deliver a different material or fluid, operate according to different characteristics (e.g., different ultrasound frequencies), or have different features (e.g., a larger or smaller ultrasound horn, different delivery options).

Another such embodiment is depicted in FIG. 1c, in which a single treatment wand 40 interfaces with or comprises a plurality of tubes 120a, 120b, 120c for delivering different fluids or materials. While three tubes 120a-c are depicted in FIG. 1c, more or fewer tubes 120 can be included in treatment wand 40 in other embodiments. Having a plurality of tubes 120 enables different fluids or materials to be delivered by a single treatment wand 40. System 10 can comprise hardware (e.g., valves, manifolds and/or pumps) along with a microprocessor and software and/or firmware to control delivery of the fluids sequentially, simultaneously (e.g., by mixing), or according to other delivery patterns. The microprocessor and other control elements and features of system 10 and device 20 are discussed in more detail herein below.

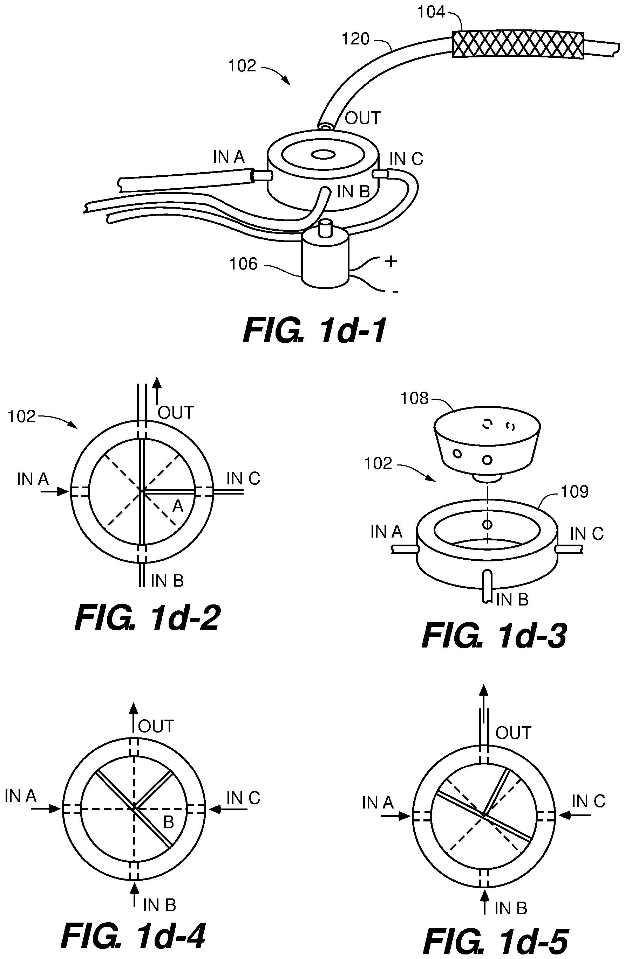

An example of a valve suitable for use with treatment wand 40 of FIG. 1c is depicted in FIG. 1d. Referring to FIG. 1d-1, an example valve 102 is depicted. Valve 102 comprises a rotary valve with stop cock-type sealing or an O-ring, though other valve types can be used in other embodiments. In some embodiments, all or a portion of valve 102 can be disposable. Valve 102 includes a static mixer 104 and can be driven by a servo motor, solenoid or stepper motor 106, or some other actuator that allows for discreet positioning of the valve.

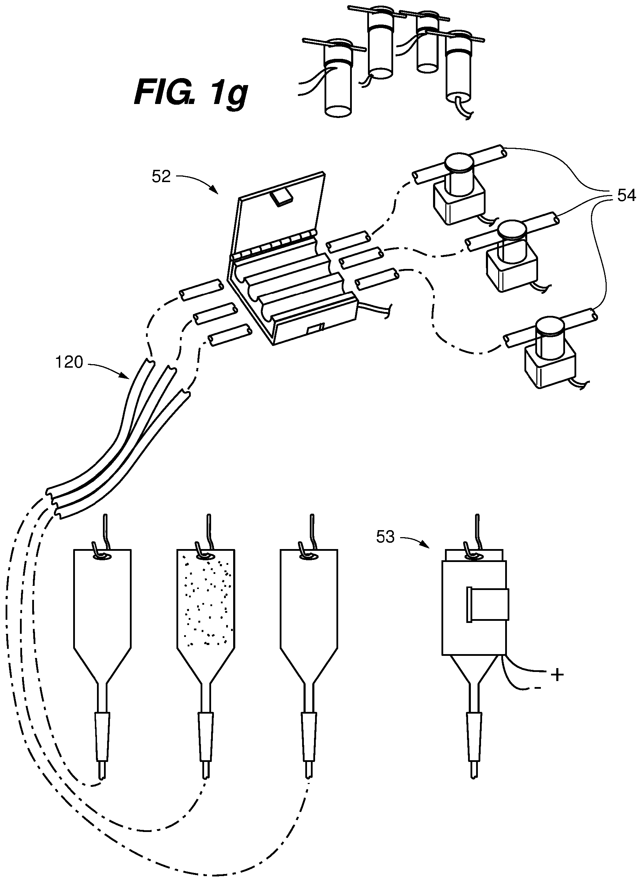

In the embodiment of FIG. 1d-1, valve 102 comprises three inputs InA, InB and InC, and one output Out. Valve 102 can be controlled manually or automatically (e.g. by a microprocessor of device 20 in generator unit 30 controlling motor 106, as discussed in more detail below) to selectively couple one or more inputs InA, InB and/or InC with output Out as depicted in FIGS. 1d-2, 1d-3, 1d-4 and 1d-5, depending on a rotational position of a center valve portion 108 within a housing portion 109 with which inputs InA, InB and InC and output Out interface. One or more pinch valves (see, e.g., FIG. 1g) can be used with valve 102 to selectively control the fluid(s) or other materials by occluding fluid flow through compression of tubing placed in the pinch valve. Like valve 102, the pinch valve(s) can be controlled by solenoids, stepper motor(s) or some other mechanical actuator. Thus, valve 102 can selectively control mixing and/or delivery of different media. In some embodiments, one media can be a cleaning or disinfecting solution. In another embodiment, one media can be a biologic or cellular material containing fluid. In yet another embodiment, the media can be a fluid for providing a protective coating, a biologic activating coating, or some other coating on a wound, ulcer or other tissue.

In some embodiments, mixing also can be accomplished using a vibrating plate, which can be incorporated in a heater block (discussed below) or separate holder. Vibration of the plate can be provided by solenoids or some other mechanical actuator, including an ultrasonic device.

While valve 102 is generally configured to deliver fluid media, in other embodiments system 10 can facilitate applying a powder, such as one comprising cellular or biologic material, directly to a wound or other patient tissue. This powder application can be followed by ultrasound delivery of saline or another fluid or material in some embodiments. In these embodiments, a different type of valve or system can be used to facilitate application of a powder.

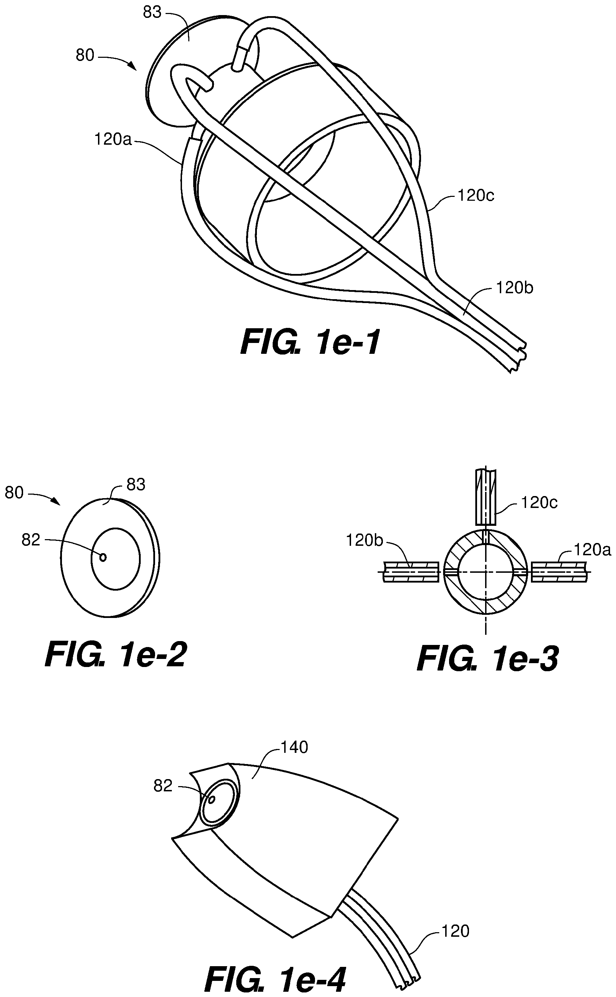

In another embodiment, and referring to FIG. 1e, a nozzle or applicator 80 can be configured for use with of treatment wand 40 of FIG. 1c. As shown in FIG. 1e, applicator 80 facilitates coupling of three tubes 120a-c with treatment wand 40 to deliver one or more fluids to tip 140 of ultrasonic transducer 70. FIG. 1e-1 is a perspective view of applicator 80, FIG. 1e-2 is an end view showing an orifice 82 of applicator 80, FIG. 1e-3 is a cross-sectional view through orifice 82, and FIG. 1e-4 is a perspective view in which a portion of a housing 84 of treatment wand 40 is included. In some embodiments, only a single fluid is delivered at any time, via one of tubes 120a-c. Thus, a plurality of fluids can be delivered sequentially or in some other order. This can be controlled by a microprocessor controlling, e.g., a series of pumps each coupled with tubes 120a-c and discussed in more detail below. In other embodiments, two or more fluids or materials can be delivered simultaneously to applicator 80 for mixing at orifice 82.

To assist the flow of fluid and help prevent clumping or aggregation of the biomaterials or other particulate, one or more coatings can be applied to the inner surface of the tubing. These coatings can comprise hydrophilic or hydrophobic coatings, such as those available commercially from Surmodics, Harlan Medical, or BioCoat. In some embodiments, the coatings can comprise specially developed or formulated material to interact with the biomaterial being dispensed.

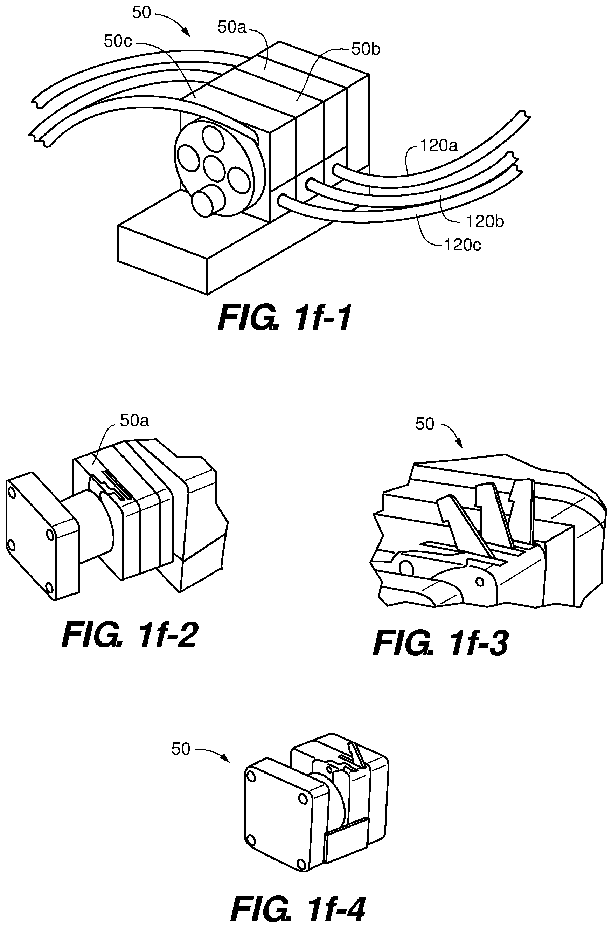

In embodiments discussed above, one or more pumps can interface with tubing 120 to control delivery of fluids to treatment wand 40. FIG. 1f depicts an example fluid pump configuration that can be used in embodiments. In particular, FIG. 1f-1 depicts a stackable head peristaltic pump 50. Pump 50 comprises a plurality of pump units 50a, 50b, 50c each arranged along or interfacing with a tubing 120a, 120b, 120c, respectively, between a fluid source (e.g., container(s) 130 discussed below) and treatment wand 40 to provide and/or control delivery of fluid or other material. In other embodiments, pump 50 can comprise more or fewer pump units, or a different type of pump (e.g., syringe or plunger pump). FIGS. 1f-2, 1f-3 and 1f-4 show additional views of pump units 50a-c.

Pump 50 can be controlled by a microprocessor of device 20, as discussed below. In operation, pump 50 can be controlled to operate one or more of pump units 50a-c at any time to deliver a fluid other material in tubing 120a-c to treatment wand 40. Pump units 50a-c can operate sequentially to deliver different materials in sequence, or one or more of pump units 50a-c can operate simultaneously to provide one or more fluids for mixing or concurrent delivery. Pump 50 can operate in conjunction with valving (e.g., valve 102) in embodiments.

Additional active or passive valving also can be incorporated into system 10 and device 20. For example, it may be desired or required to heat or cool one or more of the materials to be delivered. Referring to FIG. 1g, some embodiments of system 10 can comprise at least one temperature control unit 52. Temperature control unit 52 can comprise a Peltier effect heat/cool device, a resistive foil heater, or some other heating and/or cooling device. In some embodiments, a bottle warmer-type device 53 can be used for heating or cooling, such as on an IV bag, a sterile container, or some other source of the material to be delivered by system 10. Temperature control unit 52 can comprise one or more valves, such as pinch valves 54 or another type of valve or arrangement, for controlling fluid or material flow in tubing 120, which is arranged in or otherwise passes through temperature control unit 52. This arrangement heats or cools the material in tubing 120 as it flows or passes through temperature control unit 52. In some embodiments, a first cooling unit 52 can be provided along with a second heating unit 52. In other embodiments, a single unit 52 can provide both heating and cooling. In still other embodiments, one or more units 52 can be combined with a bottle-based temperature control device 53. Still other temperature control types and configurations also are possible, including infrared, dissipative and passive temperature control arrangements (e.g., insulative materials arranged around portions of system 10, heat generating portions of system 10 arranged around or proximate to portions of system 10 to be heated, etc.). Temperature control unit 52, device 53 and valves 54 can be controlled by a microcontroller of device 20, discussed in more detail below.

In other embodiments, valves 54 (or any other valves discussed herein) can be passive rather than active. For example, in some embodiments as already discussed the valves can be controlled by a microcontroller. In other embodiments, however, the valves can comprise springs or other mechanisms that are passive and controlled by fluid flow speed or some other property.

FIGS. 2 and 3 are high-level block diagrams of components of ultrasound therapy system 10. In general, as depicted in FIG. 2, system 10 comprises generator unit 30; treatment wand 40; fluid management pump 50; an ultrasonic driver 60; an ultrasonic transducer 70; and an applicator 80.

Generator unit 30 and treatment wand 40 are connected by a cable 90. Ultrasonic driver 60 comprises hardware mounted inside generator unit 30. A basic function of the ultrasonic driver 60 is to generate electric power output to drive ultrasonic transducer 70. Ultrasonic transducer 70 includes an acoustic horn 100 and related assembly mounted inside treatment wand 40. Ultrasonic transducer 70 converts and transfers input electrical power into vibrational mechanical (ultrasonic) energy that will be delivered to the treatment area (i.e. to a patient wound area via atomized saline). Treatment wand 40 is configured to appropriately position and hold applicator 80 relative to acoustic horn 100 for proper delivery of fluid or other another material, such as a biologic or cellular material, to be delivered during operation.

Appropriate positioning of applicator 80 also depends on appropriate positioning of treatment wand 40 by a user during use. Therefore, in embodiments treatment wand 40 can comprise a motion processing unit 42 to assist a user with proper positioning of treatment wand 40. Motion processing unit 42 can comprise at least one of an accelerometer, a gyroscope, a magnetometer and/or another device that can determine an attitude, position and/or orientation of treatment wand 40 during use. One example of a motion processing unit that may be suitable for use in system 10 is a BNO055 Application Specific Sensor Node (ASSN) available from BOSCH SENSORTEC, but this is but one example, and other units or sensors can be used in other embodiments. Motion processing unit 42 may be able to achieve levels of resolution that allow for determining treatment distance and provide more detailed information on the actual therapy to generator unit 30 and the user (i.e., treatment coverage area, distance from the skin, etc.).

Motion processing unit 42 can interface with user interface 110 (discussed below) to provide feedback or alerts to a user during operation of system 10. For example, a treatment area or patient position may lead a user to operate treatment wand 40 at an angle or in a position that interferes with proper operation (i.e., one that causes fluid to flow away from or build up on and overload transducer 70), and an audible, haptic or visual alert to a user can cause them to take corrective action and reposition treatment wand 40 relative to the treatment area. In some embodiments, an effective treatment angle between wand 40 and a treatment area is about +/- 30 degrees, and while other angles are possible, motion processing unit 42 can assist a user in maintaining a most effective position. In some embodiments, the effective or preferred angle can vary, and motion processing unit can comprise customized algorithms that correspond to different treatments, therapies, types of wounds and/or other characteristics related to treatment and assist a user in achieving or maintaining an appropriate treatment position of wand 40.

Treatment wand 40 also contains the system's user interface 110 and controls for parameters of the treatment, though in other embodiments an additional or alternative user interface can be incorporated in generator unit 30. For example, user interface 110 can provide a way for a user to select various ultrasound parameters, including frequency, intensity, pulse length, beam characteristics, and application time on the skin. Selection and customization of these parameters can be used to provide or enhance the transportation of biological cellular agents into the epidermal, dermal and subcutaneous tissues in embodiments in which materials comprising these agents are used. Further, selection and customization of these parameters can be associated with a patient characteristic, wound or type of tissue to be treated, or other factor related to treatment, in addition to the type of material to be delivered.

The configuration also provides appropriate atomization of the fluid or other media to be delivered from sterile reservoir(s) or container(s) 130 and delivery of the resulting mist and ultrasound energy to a wound or other treatment area. Sterile container(s) 130 can include one or more reservoirs containing biologic materials and various fluids. Cells or subcellular biologic materials of many types may be loaded into a liquid, for example one having the viscosity of or similar to water and placed in a sterile container 130.

A fluid containing a biologic, cellular or other biomaterial can be provided within a deliverable solution or as a separate component stored within a separate reservoir (130). A fluid containing a biologic can have a suitably high concentration such that it can be mixed to make the liquid concentration of cells homogeneous, maintain suspension of the particles of the biologic in the fluid, and ensure the fluid is ready for insertion in and application by the delivery apparatus. This mixing can be done before or at delivery, such as via an arrangement of sterile containers 130 containing the various materials and fluids for mixing, along with associated tubing and pump(s) 50 (see, e.g., FIG. 1f). This configuration, and pump(s) 50 in particular, can create a precise and controllable flow that allows for sterile delivery of the media to the patient via applicator 80 and transducer 70. In embodiments, two or more independent streams can be mixed via a static mixer (e.g., 104 in FIG. 1d-1), a vibration mixer, or other mixing device, and the streams flow rates can be independently controlled, providing a specific mixed concentration or a continuously variable rate of flow. Mixing also can be performed by one or more of the aforementioned mixing devices to prepare a single fluid for delivery (i.e., mixing a particulate material with a fluid) or to mix fluids prior to or during treatment. For example, some fluids may require vibration or other mixing of container 130 during treatment in order to maintain desired characteristics of the fluid. The mixing (e.g., type, timing) and flow rates can be set or adjusted according to a particular material to be provided, therapy to be delivered, patient characteristic, or other factor(s).

In other embodiments, these mixings and delivery methodologies can be accomplished by mixers, as previously mentioned, and/or flow channel and valving configurations. In one embodiment, these hardware components are provided with or coupled to pump(s) 50. For example, generator unit 30 and/or treatment wand unit 40 can open and close various valves (e.g., each valve associated with a container 130 or other supply of fluid or material) during operation to provide mixing, staged delivery of different fluids, materials, or concentrations, other techniques. Refer, for example, to FIGS. 1d, 1e and 1f.

For example, in one embodiment a first fluid is delivered to clean or disinfect the wound or other patient tissue, then a second material for additional cleaning or preparation can be delivered, then a third material comprising the desired biologic material. Finally, a fourth or last material can be delivered to provide a protective or other coating on the tissue (e.g., to activate the cells and provide a barrier). The valving and/or pumping to accomplish this can be automatic according to a programming of settings, which in one embodiment itself can be automatic according to a scanning of a machine-readable code (discussed below) or manual by user programmable settings.

In other examples, a biologic fluid is delivered prior to saline and ultrasound treatment. In another embodiment, the biologic fluid is delivered simultaneously with saline and ultrasound therapy. In these or any other case, system 10 can be automatically or manually set, adjusted and/or used according to the desired delivery and treatment or therapy type.

In some embodiments, surfaces that the fluid comes into contact with (e.g., within containers 130, pumps 50, valve 102, tubing 120, applicator 80, etc.) can be coated with various compounds or materials that either positively interact with the fluid or provide a neutral surface that does not interact with the material. For example, the coating can contain a component material that activates the cells as they come in contact with the coating. The coating can be dry or wet.

In some embodiments, the solution can be mixed and placed into a single use sterile container. This container can be directly coupled to as container 130 in some embodiments, or this container can interface with container 130 (e.g., be placed within a holding container 130) in system 10. Given the cellular and biologic nature of the material in some embodiments, the container can be specially marked to ensure use with the proper patient. For example, bar coding, Quick Response (QR) coding, radio frequency identification (RFID) tagging, or other wirelessly, electrically or mechanically readable technologies can be used to label the container. System 10 can include corresponding reading technology (e.g., a bar code reader, an RFID reader, etc.), such that system 10 can require that a patient identification (e.g., wrist band, medical chart, biometric identifier, etc.) is read, then the corresponding container is read and confirmed to match the patient before treatment via system 10 can begin. System 10 can additionally or alternatively confirm that the fluid is for use with a particular patient; confirm a content of the reservoir; or receive device programming information from the machine-readable label. In other embodiments, system 10 can comprise a reader configured to read a machine-readable component, such as a bar code, QR code, RFID tag, or the like, on a component or accessory of system 10 to confirm that the component or accessory is usable with system 10.

This scanning also can provide programming information for operation of system 10, which then can be automatically set by system 10. In some embodiments, user confirmation of these settings can be required, or some manual entering or interaction with system 10 can be implemented. The settings can include intensity, intensity variation via electronic controlling signal (wave shaping), flow rate(s) of fluid(s), duty cycle of the output, variations in frequency within a defined range (i.e., large variation can require a separate transducer 70 to resonate at the desired frequency, and in embodiments system 10 can accommodate various different transducers 70), and other settings. For example, in one embodiment saline is delivered first at a first, lower intensity, then the cellular material is delivered via a second, higher intensity. During delivery of the cellular material, the intensity can be further adjusted (e.g., increased or decreased) so that treatment ends at a third, even higher intensity. In embodiments, settings, including those made automatically, can be adjusted on the fly during operation, such as in the case of patient discomfort with or intolerance of a higher frequency or other setting. In one embodiment, this can be done via user interface 110. In another embodiment, this can be done via treatment wand unit 40, which instead of providing a two-state, on/off trigger can comprise variable or proportional trigger that increases or decreases with displacement thereof by the user (i.e., more force applied to or displacement of the trigger applied by a user increases intensity or rate of fluid delivery, etc., while less force or displacement decreases intensity, rate of fluid delivery, etc.). In some embodiments, the characteristic that is variable in this manner is user-selectable via user interface 110, while in other embodiments the characteristic is automatically selected or set.

In some embodiments, the machine-reading components can be provided in treatment wand unit 40. In other embodiments, these components can be implemented elsewhere in system 10, such as generator unit 30.

In some aspects the biologic or cellular material containing fluid may be provided in kit or as a separate component from saline or other fluids. In one embodiment, the kit can comprise one or more sterile containers 130 or containers that can be coupled to or with sterile containers 130 and/or pumps 50 and comprising a fluid or material for mixing and/or delivery; tubing 120 for coupling pumps 50 and applicator 80; and applicator 80. In such an embodiment, tubing 120 and applicator 80 can be single use and disposable, which can be required or convenient when the fluid delivered by these components includes biologic or cellular material. In other embodiments, the kit can additionally or instead comprise a detergent or cleaning agent, such as one provided in a container 130 for coupling with and running through system 10 after use with a biologic or cellular material to ensure no material remains in system 10 for use with the next patient. Other cleaning methodologies can be used in other embodiments and can comprise manual or automatic cleanings.

In other embodiments, kits can additionally or instead comprise disposables to form a disposable kit, such as a single-use applicator 80 coupleable to treatment wand 40, tubing to convey the fluid from container 130 to treatment wand 40, and a spike to pierce container 130 to couple the tubing to container 130. A kit can additionally comprise at least one of a valve or a disposable mixer. Kits of disposables can be convenient for facilities using system 10 while also reducing the risk of cross-contamination of system components and/or reuse of components with multiple patients.

Fluid management pump(s) 50 (see also FIG. 1f) can provide a fixed flow rate of saline or other fluid and biologic and cellular materials for delivery (i.e., "the material for delivery" will be used herein generally and can comprise any of the fluids, biologic/cellular materials, or other liquids, powders, suspensions, gases, or other materials, as discussed herein) via tube(s) 120 to the distal tip 140 of ultrasonic transducer 70 from one or more sterile container(s) 130 and/or fluid reservoirs or other sources, as appropriate. Container(s) 130 can comprise one or more bags, bottles, packs, cups, or other packages suitable for holding the material for delivery and coupling with or in system 10. The material in container(s) 130 is delivered to the radial surface of transducer horn near its tip 140 by one or more tube(s) 120 and applicator 80. The material is dispensed through an orifice (e.g., 82 in FIG. 1e) on a superior surface of the horn 100, and a portion of the material is displaced forward to the face of horn 100 and atomized by horn 100 when horn 100 is energized and operating. The remaining volume of material for delivery is fed to an inferior surface of ultrasonic transducer 70 via gravity and capillary action. When a sufficient volume of material is accumulated, transducer tip 140 atomizes the material into a plume. The atomized material spray plume emanates from two points on the ultrasonic transducer 70, i.e., generally at the 12 o'clock and 6 o'clock positions given normal positioning of treatment wand 40 in operation, forming intersecting spray paths at approximately 5 mm from the front face of ultrasonic transducer 70 in some embodiments. Depending on the location of orifice(s) 82, the spray plume may be varied or modified. While single-point dispensing has been discussed, multi-point dispensing also can be used in some embodiments. Additionally, the configuration of the shroud 83 (see FIG. 1e) also can contribute to controlling the spray plume, as shroud 83 acts as a reflective surface. In other embodiments, treatment wand 40, transducer 70, orifice 82, horn 100, tip 140, applicator 80 and/or other components can be designed to provide a differently sized or configured spray plume and paths, such as one customized for a particular material to be delivered, wound or patient anatomy to be treated, therapy to provided, or other characteristic. In still other embodiments, these components of device 20 can vary in size or other characteristics, and a user can select (or device 20 can prescribe) a particular combination of the components, selectable from a kit comprising the components with various characteristics, to use with device 20 according to a particular treatment or therapy to be provided, a fluid to be delivered, a patient or condition to be treated, or some other factor. Instead of or in addition to varying the physical characteristics or specifications of one or more components of device 20, one or more operating characteristics can be varied in order to alter the spray pattern, such as the flow rate, frequency, angle of delivery, delivery distance, delivery pattern (e.g., the way in which a user manipulates wand 40 during treatment), or some other characteristic.

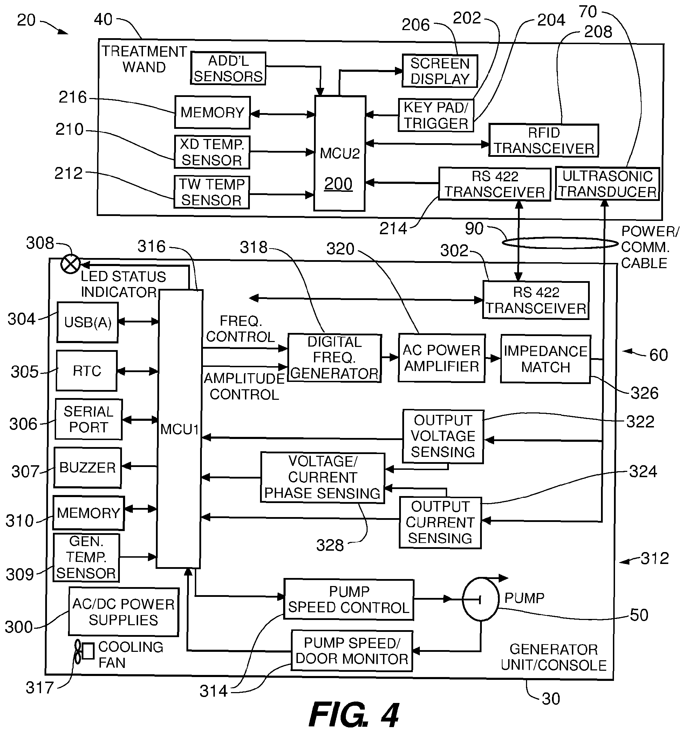

FIG. 4 is a block diagram of a more detailed schematic of generator unit 30 and treatment wand 40 of ultrasound device 20. As can be understood from the following description, parameter control of voltage, current, duty cycle and phase angle is enabled, in some embodiments.

Treatment wand 40 houses ultrasonic transducer 70 and includes a microprocessor 200, various interface and sensing components, and an OLED display 206. Treatment wand 40 is pistol-shaped in embodiments to provide an improved ergonomic design, though other configurations can be implemented as may be advantageous in some applications. Treatment wand 40 comprises an acoustic horn assembly (e.g., piezo elements, back mass, horn and booster), ultrasonic transducer 70, microprocessor (MCU2) 200, user control key pad 202 and trigger 204, and an LCD screen display 206 that displays operational information and enables control and programming of the treatment therapy (see, e.g., FIG. 1). Treatment wand 40 also includes an RFID transceiver 208 in some embodiments, and RFID transceiver 208 can be used to identify applicator 80. This feature can be used to ensure that there is only a single use of a particular applicator 80 and to thereby deter unwanted reuse across multiple patients and/or treatments. This feature can be particularly advantageous in embodiments in which patient-specific cellular and biologic materials, information, data and/or characteristics are used in system 10. Thus, in embodiments the RFID chips read by transceiver 208 can contain information about the specific treatment, which is conveyed to system 10 by this reading, providing inputs for the specific therapy to be administered by device 20.

Treatment wand 40 connects to generator unit 30 through cable 90. Cable 90 includes ultrasonic driver output power, RS488 communication and +5V power in an embodiment, though other power and/or communications features can be implemented or facilitated by cable 90 in other embodiments. In one embodiment, 3.3V and 13V power will be generated from the 5V power provided by generator unit 30 for the electronics in the treatment wand 40. These example power characteristics can vary and are merely examples of one embodiment.

User interface 110 on treatment wand 40 includes key pad 202, trigger 204, and screen display 206. In some embodiments, display 206 can be a full-color OLED display, and key pad 202 can be a four button display, as shown in FIG. 1. The operator can configure and control device and system operation via key pad 202 and initiate the delivery of therapies by depressing a trigger switch 204.

Microprocessor 200 that controls user input requirements can also measure the internal temperature of treatment wand 40, or of transducer 70 or horn 100 more specifically, from ultrasonic transducer sensor 210 and treatment wand temperature sensor 212. In embodiments, temperature sensors 210 and/or 212 or additional temperature sensors in treatment wand 40 or elsewhere in system 10 can be used to provide active thermal control. For example, in embodiments the material to be delivered (e.g. from container(s) 130) can be heated or cooled for delivery. Temperature sensors throughout system 10 can monitor the temperature of the material at various places in system 10 (e.g., in treatment wand 40 by sensor 212, and/or at container(s) 130, pump(s) 50, tube(s) 120 and elsewhere by additional sensors at those places) to ensure it is as desired. For example, in one embodiment, a sensor can monitor a temperature of the tissue, and system 10 can adjust the fluid dispensing temperature as needed. In some embodiments, heating or cooling elements can be provided, such as along tubing 120 and/or around container(s) 130. In still other embodiments, heat generated during normal operation of system 10 can be used to heat the material, and/or this heat can be dissipated by cooling effects of the material, such as via selective arrangements of tubing 120 proximate heat sources. Still other sensors can be incorporated in or communicatively (e.g., wirelessly) coupled to treatment wand 40 for assessing and monitoring the tissue during operation of system 10. Based on this assessing and monitoring, system 10 can adjust or regulate the therapy being provided. For example, wand 40 can comprise an infrared sensor to sense patient tissue temperature during treatment and provide feedback to an operator, who can adjust settings to provide heating or cooling or adjust other settings to increase patient comfort and therapeutic response. Still other types of non-contact sensing - or contact, such as via wireless sensors applied to a patient's skin - can be used in other embodiments, including imaging techniques, which can provide information regarding the size of a wound or lesion for use in determining optimum settings and therapies. Microprocessor 200, or another processor in system 10, can monitor and control these sensors and elements in operation, in various embodiments.

Microprocessor 200 also sends read/write information to applicator 80. Microprocessor 200 communicates with generator unit 30 via an RS488 transceiver 214 and writes information to EEPROM 216. This information is stored and can be retrieved for understanding the use and performance of the system. Accordingly, greater detail can be given on data stored, how much, how long and how retrieved (USB upload/download by the user, service or other).

RFID transceiver 208 of treatment wand 40 can be used to communicate with an RFID tag (not shown) for applicator detection, as previously mentioned. The RFID tag can be located on applicator 80, and microprocessor 200 in treatment wand 40 can serve as an RFID reader and writer of the signals received via RFID transceiver 208. Specifically, an RFID controller can be used in treatment wand 40 for a Read/Write RF tag on applicator 80 and/or container(s) 130. In each new treatment, system 10 will require a new applicator 80 and container(s) 130. The RFID controller can read the ID tag of applicator 80 and container(s) 130, as well as a patient identifier on a bracelet, chart or other location, to identify if that particular applicator 80 is new or used, whether that applicator 80 is suitable for use with the material in container(s) 130 (e.g., according to particle and aperture sizes), and/or whether the material in container(s) 130 is suitable for the particular patient to be treated. The RFID controller also can obtain information related to specifics of the biologic or cellular material in container(s) 130, a suitable or suggested therapy for use with that material or for a patient condition, time usage, and other therapeutic and treatment characteristics. For example, in one embodiment the RFID controller can read diagnostic information, obtain usage or other information about a disposable (e.g., applicator 80), and use this data and information for system review and diagnostics if a user reports errors or problems, if a device or disposable is returned as being inoperable or defection, or for other purposes. In embodiments, the RFID controller also can write information to the tag for treatment monitoring and reporting. After a particular applicator 80 is used for a specified period of time, the RFID controller can write the information to an ID tag to identify that applicator 80 has been used to avoid reuse. As previously mentioned, treatment wand 40 also can comprise other machine-readable hardware (e.g., bar code reader), instead of or in additional to RFID transceiver 208, and bar codes or other machine readable devices can be arranged on other components of system 10.

Microprocessor 200 of treatment wand 40 can be used to control all inputs and output functions and perform all control loops, and calculations. Features of some embodiments can include: an 80 MHz maximum frequency; 1.56 DMIPS/MHz (Dhrystone 2.1) performance; an operating voltage range of 2.3V to 3.6V; a 512K flash memory (plus an additional 12 KB of Boot Flash); a 128K SRAM memory; a USB 2.0-compliant full-speed device and On-The-Go (OTG) controller; up to 16-channel, 10-bit Analog-to-Digital Converter; six UART modules with RS-232, RS-485 and LIN support; and up to four SPI modules. These features are merely examples of one embodiment and can vary in other embodiments.

Ultrasonic transducer 70 generally comprises a piezoelectric ceramic element and metal horn 100 mounted in a sealed housing. The ultrasonic transducer input can be an AC voltage or AC current, and the waveform can be a square form or sine form. The ultrasonic transducer output is mechanical vibration of the tip of transducer 70. The amount of energy output depends on tip 70 displacement, operation frequency, size and driver load (e.g., air or liquid mist). The ratio of output to input energy is referred to as the electromechanical coupling factor. There are many variables that affect coupling factor, including operation frequency. In theory, it would be advantageous to operate an ultrasound transducer (UST) by keeping the operating frequency in the resonance frequency (Fr) or anti-resonance frequency (Fa) region because its electrical power factor is 1. However, due to the related, very unique impedance-frequency characteristics of these transducers, which can vary from transducer to transducer, drive circuit design is very difficult. In previously designed ultrasonic drivers, Phase Loop Lock (PLL) techniques were widely used. Because of the nature of analog performance, keeping a highly accurate and stable frequency output was very difficult. In theory, a UST that operates at Fr or Fa has a high efficiency output. In practice, operating a UST at Fr or Fa is almost impossible with PLL technology. This is why most ultrasonic drivers with a PLL design only can operate in Fr or Fa regions rather than at Fr or Fa points, and the operational phase typically must be more than 50 degrees. For most systems with rapidly changing load impedance, operation at frequencies close to Fa or Fr will cause the system to be unstable. Alternatively, a system can be kept running stable by setting the operation frequency lower than Fr or higher than Fa points, as in past designs. In embodiments discussed herein, however, the ultrasonic driver can be monitored and controlled to operate at or very near Fr, a significant advantage over conventional systems.

Ultrasonic transducer 70 is operated at relatively large displacements and a low load condition, thereby reducing loading effects and electrical impedance. Accordingly, ultrasonic medical applications use a constant current control algorithm because of the following performance advantages: increased electrical safety due to lower operating voltage; proportional current to tip velocity (displacement if frequency is held constant); and the capability to limit excessive power surges by setting the voltage rail to an appropriate value, among others.

As discussed elsewhere herein, in embodiments system 10 can be compatible with or comprise more than one ultrasound transducer 70. This plurality of USTs 70 can be operated sequentially or even simultaneously in embodiments, or an operator can select a single UST 70 from among the plurality for use in system 10. In embodiments, microprocessors 200 and/or 316 can automatically adjust for a selected UST 70 or multiple USTs 70, which can be implemented in multiple treatment wands 40 in embodiments.

Generator unit 30 includes a power entry module and AC/DC power supply 300 as well as an ultrasonic driver 60. Delivery pump(s) 50 are mounted on generator unit 30 in one embodiment and are controlled by a pump driver located on ultrasonic driver 60. In another embodiment, a pump(s) 50 can be provided in a separate pumping unit, such as one that additionally couples with or comprises mixing and valving components as previously discussed. Communications ports 302, 304, and 306 are also located on the generator unit 30, though the number and arrangement of communications ports can vary from those depicted. For example, in other embodiments more or fewer ports are provided, and one or more of the ports can comprise a wireless communications port (e.g., infrared, RF, BLUETOOTH, WIFI or some other wireless technology). These ports provide an information exchange between generator unit 30 and treatment wand 40 as well as information exchange between device 20 and user.

With respect to the Power Entry Module & AC/DC Power Input, in some embodiments the local AC MAINS is connected to an appliance inlet with a hospital grade detachable power cord. In some embodiments, two power cords will be used, 15A with a 125V rate and 10A with a 250V rate. In some embodiments, the appliance inlet is a power entry module listed for medical applications with an 10A current rating, 120/250VAC voltage input, MAINS switch, integral fuse holder (2 ¼ x1 1/4" / 5x20mm fuses), EMC line filter for medical applications, and is mounted on the rear panel of the chassis. Although not depicted in the figures, embodiments are contemplated that use battery power as the power source in the system's design. The battery would be located within generator 30 in various embodiments. Battery power is made possible due to the extremely efficient design discussed herein.

In some embodiments, system 10 can have a universal AC power input capability accepting a range of power input from 90V to 265VAC. The local AC MAINS are connected to an appliance inlet component (IEC 320 C14) with a hospital grade detachable power cord. The appliance inlet is a power entry module listed for medical applications with an 115V / 230V voltage input, MAINS switch, integral fuse holder (2 -5x20mm fuses), and an EMC line filter for medical applications that is mounted on the rear panel of the chassis. The MAINS switch output is connected to two AC/DC switching power modules. The two AC/DC (24V output) switching power supply modules are serially connected together to provide +/-24V power to AB type amplifier use. All DC power sources +5V, +4.5V, -4.5V and 3.3V are generated from +24VDC - power source via DC/DC converter. The +5VDC will provide 5V power to treatment wand 40 through the detached cable and medical grade connector 90.

In some embodiments, two identical AC/DC (24V output) switching power supply modules are serially connected together to provide +/-24V power to AB type amplifier use. The power supply can be medical grade, Class II, BF rated with 45W output with conventional cooling. A dual color (Red/Green) LED 308 can be mounted at the front of generator unit 30. The green color indicates normal power on without errors, and the red color indicates a system error or failure. Error detail information can also display on the interface display screen 206 of treatment wand 40.

In some embodiments, there is a plurality of, such as three, communication ports in the on generator 30. The first port is a RS488 communication port 302, with 5V power and XD outputs. This port 302 is connected to treatment wand 40 through cable 90. Port 302 can be configured for full duplex communications in both directions at the same time. This port 302 can handle information exchange between generator unit 30 and treatment wand 40. In operation, both sets of microcontrollers 200 and 316 can check each other to ensure none has failed to operate through this port 302. The second port can be a USB-2 type A port, referred to herein as port 304. It can be designed for user download of information stored at the EEPROM memory 310 by using flash key device. This port 304 can be used for uploading software from flash key device. A third port can be an RS232-3.3V serial port, referred to herein as port 306. Port 306 can be designed for use with a PC, so the PC can communicate to the system 10 for download, upload, system debug and calibration. Also included in generator unit 30 and connected to the microcontroller are RTC DS1306 at numeral 305, audible signal generator 307 and generator temperature sensor 309. Additional sensors can be included in generator unit 30 in other embodiments, as discussed elsewhere herein.

A microcontroller controlled pump delivery system 312 can be used for fluid and material delivery. Delivery system 312 comprises pump(s) 50 and pump driver with controls 314 for pump speed and pump doors monitoring and can deliver fluid, such as saline or another fluid enriched with a biologic or cellular material, through a tube 120 and applicator 80 to the tip of ultrasonic transducer 70. Microcontroller (MCU1) 316 of generator unit 30 can control peristaltic pump speed to control fluid flow rate for a fixed tubing size. Pump delivery system 312 generally operates at constant flow rate for all operating conditions. A cooling fan 317 is mounted in the back of generator unit 30. It is controlled by microcontroller 316 of ultrasonic driver 60.

Ultrasonic driver 60 includes a microprocessor 316 that controls, measures and monitors the drive electronics and communicates with the hardware and software of the treatment wand 40. In some embodiments, ultrasonic driver 60 includes a microprocessor 316 (such as Microchip Technology Inc. PIC32) with an 80MHz clock and 1.56DMIPS/MHz performance, though some other suitable microprocessor can be used in other embodiments. The drive electronics contain a digital frequency generator (DDS) 318, AC amplifier 320 and voltage and current phase detection circuits 322 and 324. Digital frequency generator 318 generates accurate frequencies set by microprocessor 316 to AC amplifier 320 that are output via impedance match 326 to ultrasonic transducer 70. Voltage and current phase detection circuits 322 and 324 continually monitor the phase difference sensed at 328. In operation, microprocessor 316 can adjust the digital frequency generator output frequency based on voltage and current phase angle so that the frequency is locked at the resonance frequency Fr of ultrasonic transducer 70. The resonance frequency Fr is not a fixed frequency, however, as it can drift with temperature and other changes. This is discussed herein below in additional detail.

Ultrasonic driver 60 also includes a digital frequency generator 318, a resonance frequency control loop 400, and an output current control loop 500. Microcontroller 316 can be of sufficiently high speed so as to handle all input measures and output settings, especially for phase comparison of cycle by cycle frequency adjustment in real time. Ultrasonic driver 60 generates electrical output with an ultrasonic frequency and a required power.

At Fr and Fa, the impedance phase is 0 degrees, which means that ultrasonic transducer 70 can achieve the highest power efficiency at those points. Accordingly, it is recognized that keeping the output frequency close to Fr or Fa would be desirable, if possible. However, it is very difficult for any control systems to operate at Fr and Fa, as at those points any small increase or decrease of frequency will cause a large impedance increase or decrease. Accordingly, most ultrasonic drivers either operate at frequencies higher than Fa or lower than Fr because frequencies are relatively stable when they are farther from Fr or Fa.

For example, some conventional systems have been designed to operate in the Fa region. These designs were relatively stable and delivered effective treatment, but output power efficiency was very low and a very high operating voltage was required. Accordingly, in order to meet regulatory safety requirements, wires with high isolation and earth protection were required, adding cost and restricted user ergonomics due to a stiffer and heavier cable.

An example comparing the voltage required by a past device operating at Fa compared to an embodiment of the currently disclosed system, operating at Fr, is set forth below:

A conventional ultrasonic transducer was operated at anti-ultrasonic region which is approximately 1KΩ~8KΩ impedance. To deliver the required power to the transducer the driver must output very high voltage (300V) to the transducer. The power calculation is:

$$P = I^2 Z * Cos\varphi \text{-----------------------------------------Equation 1}$$

P: input power of transducer
I: input current
Z: transducer impedance in Ohm
φ: voltage/current phase angle (-90°~+90°)

If the transducer requires 7W power, φ =85°, Z=1500Ω, from Equation 1 the current will be:

$$I = \sqrt[2]{\frac{P}{Z * Cos\varphi}} = \sqrt[2]{\frac{7W}{1500\Omega * Cos(85°)}} = 230mA$$

Accordingly, a power supply voltage would be: (230mA* 1500Ω) = 347V.

An embodiment of system 10, in contrast, operates at Fr with constant current output control. Its impedance is about 25-80 Ω and voltage current phase angle close to 0 degrees. The power efficiency is almost 100%. An example with Fr impedance is 50Ω.

If the transducer requires 7W power, φ =0°, Z=50Ω, the current will be:

$$I = \sqrt[2]{\frac{P}{Z * Cos\varphi}} = \sqrt[2]{\frac{7W}{50\Omega * Cos(0°)}} = 370mA$$

and the power voltage will be: 370mA *50Ω = 18.7V

Accordingly, embodiments of system 10, with a low voltage operation condition, can be much more efficient and safer than conventional designs. Any voltage surges resulting when transducer impedance is increased can be limited by setting the voltage rail to an appropriate value.

Microcontroller 316 of the ultrasonic driver controls all input and output functions and performs all control loops and calculations. Certain embodiments of microcontroller 316 may include one or more of the following: a 80 MHz maximum frequency; 1.56 DMIPS/MHz (Dhrystone 2.1) performance; an operating voltage range of 2.3V to 3.6V; a 512K flash memory (plus an additional 12 KB of Boot Flash); a 128K SRAM memory; USB 2.0-compliant full-speed device and On-The-Go (OTG) controller; up to 16-channel, 10-bit Analog-to-Digital Converter; six UART modules with RS-232, RS-485 and LIN support; and up to four SPI modules. These characteristics are merely examples and can vary in other embodiments.

The ultrasonic frequency generator is a digital frequency generator 318 that provides numerous advantages over conventional designs. In some conventional designs, PLL technology was used with a voltage control oscillator (VCO) for generating a fixed ultrasonic frequency. However, this produced an output frequency that is low resolution and not flexible for wide frequency range applications without hardware changes. Further, the frequency stability was imprecise since the VCO is affected by temperature, noise and power ripple.

In the current ultrasonic therapy system 10, a Direct Digital Synthesis programmable frequency generator (DDS) is used as part of the frequency generator 318. Because a DDS is digitally programmable, the phase and frequency of waveform can be easily adjusted without the need to change the external components that would normally need to be changed when using traditional analog-programmed waveform generators. DDS permits simple adjustments of frequency in real time to locate resonance frequencies or compensate for temperature drift. The output frequency can be monitored and continually adjusted by microcontroller 316 at real time speed. Advantages of using DDS to generate frequency include: digitally controlled micro-hertz frequency-tuning and sub-degree phase-tuning capability; extremely fast speed in tuning output frequency (or phase); and phase-continuous frequency hops with no overshoot/undershoot or analog-related loop setting-time anomalies, among others.

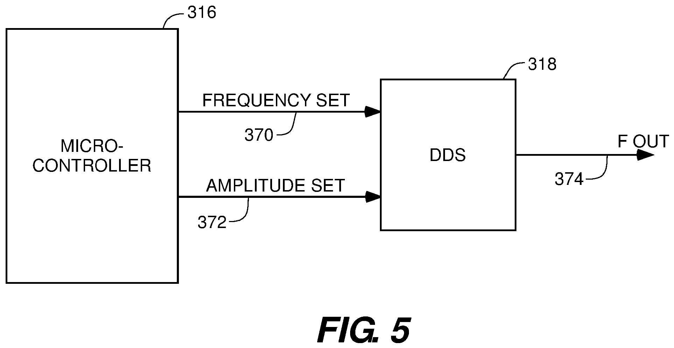

The digital architecture of DDS eliminates the need for the manual tuning and tweaking related to components aging and temperature drift in analog synthesizer solutions, and the digital control interface of the DDS architecture facilitates an environment where systems can be remotely controlled and optimized with high resolution under processor control. FIG. 5 shows the system's digital frequency generation using microcontroller 316 and DDS 318. Specifically, frequency set 370 and amplitude set 372 are received by DDS 318 which generates an output frequency 374 ($f_{out}$).

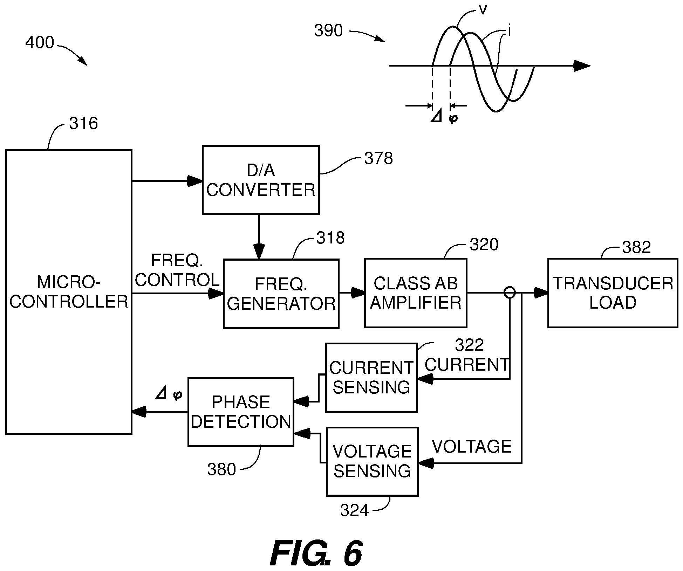

FIG. 6 sets forth the frequency control loop 400 for system 10. Frequency control loop 400 includes a digital frequency generator (DDS) 318, D/A converter 378, phase detector 380 and microprocessor 316. The drive electronics utilize the digital frequency generator 318, AC amplifier 320 and voltage and current phase detection circuits 322 and 324. Digital frequency generator 318 generates a high accuracy and precision frequency signal, set by the microprocessor 316, to AC amplifier 320 that outputs across a transducer load 382 to ultrasonic transducer 70. At start-up, system 10 performs a Power On Self-Test (POST) and communicates with ultrasonic transducer 70 to gather information on characteristics of ultrasonic transducer 70 and determine that treatment wand 40 is functioning properly.

Specifically, when initially energized, microprocessor 316 can be programmed to perform a frequency sweep using a sine wave to determine the resonant frequency by evaluating and looking for a relative minimum impedance of ultrasonic transducer 70. The sweep is confined to a smaller defined interval based on the information embedded in treatment wand 40 regarding the operating characteristics of ultrasonic transducer 70. This includes the information stored in ultrasonic transducer 70 at the time of manufacture or otherwise programmed or updated. During the system start, digital frequency generator 318 can scan frequencies from a start frequency (min 20 KHz, adjustable) to an end frequency (max 50 KHz, adjustable) to find the resonance frequency (Fr). Microprocessor 316 can adjust the digital frequency generator output frequency based on voltage and current phase angle so that the frequency lockup is maintained at the resonance frequency of ultrasonic transducer 70 (i.e., at a 0° phase angle). Because the frequencies continually shift due to temperature change and other factors, the phase of output voltage and current will change as well. The voltage and current phase detection circuits are continually monitored for the phase difference and adjusted accordingly. Resonance frequency is not a fixed frequency. This is due to heating and other factors causing a slight drift change with temperature. Specifically, increased temperature can cause decreased resonant frequency.

In order to keep output frequency lockup at resonance frequency, frequency control loop 400 can operate at the real time monitoring output voltage and current phase angle and continually adjust operating frequency to match the current resonance frequency. In some embodiments, microprocessor 316 can maintain $\Delta\varphi$ (as illustrated at 390) to less than about 0.1 degree inaccuracy and provide sufficient capabilities to achieve accuracy of about 0.1Hz or better. In some embodiments, resonance frequency is digitally controlled to better than about 0.5 Hz while maintaining constant energy output.

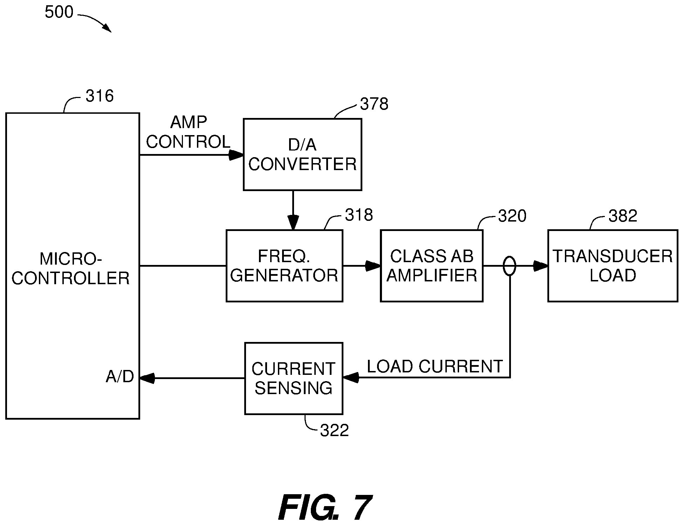

FIG. 7 sets forth output current control loop 500 for system 10. Output current control loop 500 is designed to provide a constant current output. Since the transducer output displacement is a function of transducer current, the control output current (not voltage) will control output displacement. Displacement determines the amount of ultrasound energy delivered/output. Microprocessor 316 monitors the output current via a sensing resistor then adjusts the digital frequency generator 318 output signal level to maintain constant current output thus maintaining a constant output displacement from the tip of the horn. Current sensing circuit 322 will sense peak current, then convert peak value to an RMS value. Any waveform distortion will cause converter errors causing current control errors and ultimately displacement errors. To avoid this situation, embodiments of the system can use RMS sensing technology to reduce the errors. This may be implemented if the waveform has considerable distortion, for example.

In system 10, the digital frequency generator 318 can be used to allow for selection and use of different frequencies via software implementation. Configurations having frequencies ranging from about 20-50kHz are possible. Digital frequency generator 318 is digitally programmable. Accordingly, the phase and frequency of a waveform can be easily adjusted without the need to change hardware (frequency generating components), as would normally be required to change when using traditional analog-programmed waveform generators. Digital frequency generator 318 permits simple adjustments of frequency in real time to locate resonance frequencies or compensate for temperature drift or other deviations in the resonant frequency. The output frequency can be monitored and continually adjusted by microcontroller 316 at real time speed.

There are many advantages to using digital frequency generator 318 to generate frequency. For example, this provides a digitally controlled, 0.1-Hertz frequency-tuning and sub-degree phase-tuning capability as well as extremely fast speed in tuning output frequency (or phase). The digital frequency generator 318 also provides phase-continuous frequency loops with no overshoot/undershoot or analog-related loop setting-time anomalies. The digital architecture of the digital frequency generator 318 eliminates the need for the manual tuning and tweaking related to components aging and temperature drift in analog synthesizer solutions, and the digital control interface of the digital frequency generator architecture facilitates an environment where systems can be remotely controlled and optimized with high resolution under processor control.

In this system, ultrasonic driver 60 outputs a sine waveform through a class AB power amplifier 320. It can operate at frequency from 20 KHz to 50 KHz, constant current mode. The ultrasonic driver 60 outputs current from 0 to 0.5A, voltage from 0 to 30Vrms, Max power to 15W. The ultrasonic driver output can scan resonance frequencies from the 20 KHz to 50 KHz range, detect minimum impedance (0° degree phase angle of voltage and current), and then lock operational frequency to resonance frequency of the ultrasonic transducer 70 at a ±0.5Hz accuracy level. Parameters may vary in various embodiments. In certain embodiments, the drive voltage requirements are less than 50Vrms for the system.

The technology of system 10 is unique in that sees an essentially constant load. The no-load condition is similar to the operational load. Being a non-contact treatment and dispensing only a small amount of fluid onto the horn does not create a significant variation in the load/output allowing the system to be run at resonance (Fr). Running and controlling the system at Fr allows greater efficiency, as previously discussed. Typical ultrasound applications such as welding, mixing, cutting, and cleaning have significant variation in the load, e.g., going from a no-load to full load condition. The variation makes control of the output very difficult and requires greater power at the cost of efficiency.

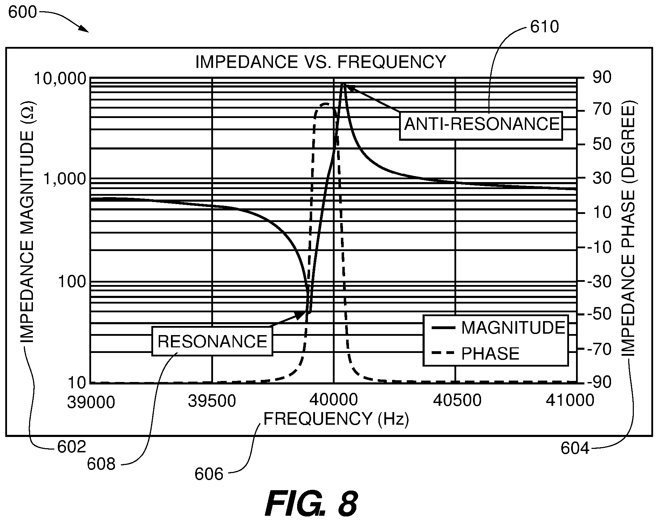

FIG. 8 is a graph that helps to illustrate advantages of using system 10's ultrasonic driver based on the impedance and frequency characteristics of ultrasonic transducer 70. Specifically, the dramatic change in impedance magnitude 602 and phase 604 is seen for changes in frequency 606 for even small deviations from the resonance frequency 608 and anti-resonance frequency 610. Ultrasonic transducer 70 is a component that converts electrical energy to mechanical energy. Its impedance and frequency characteristics create significant drive circuit design challenges, especially if trying to optimize for low power input and accuracy. Traditional ultrasonic driver designs typically use Phase Loop Lock (PLL) frequency control technology. However, analog system performance generally does not allow for accuracy and stable frequency output. Accordingly, make it difficult to control the system precisely with analog systems. In theory, an ultrasonic transducer operating at resonance frequency Fr or anti-resonance Fa frequency has a high efficiency output. In practice, when ultrasonic transducers operate at resonance frequency or anti-resonance frequency, it is almost impossible using PLL technology to maintain elegant control. Most other ultrasonic drivers utilize an analog PLL based design for control. The PLL based designs operate close to resonance frequency or anti-resonance frequency points, but due to their inherent inaccuracy, these often operate at some phase angle away from Fr or Fa leading to inefficiencies.

In system 10, a constant current control algorithm can be used. It can operate at resonance frequency, rather than just close to resonant frequency. The difference between anti-resonance and resonance is anti-resonance with highest impedance and resonance with lowest impedance. The high impedance can be range at 5KΩ~50KΩ and lowest impedance can be at 20Ω~100Ω in certain embodiments, for example.

Since ultrasonic transducer 70 is operated with relatively large displacements and a low load condition, there is a significant reduction in loading effects and electrical impedance variation. Many ultrasonic medical applications use a constant current control algorithm because of the following performance advantages: electrical safety (due to a lower operating voltage); current that is proportional to tip velocity (displacement if frequency is held constant); and fewer excessive power surges (by setting and maintaining the voltage rail to an appropriate value).

In some embodiments, and referring again to FIG. 1b, system 10 can comprise more than one treatment wand 40, and microprocessor 316 can be programmed to sequentially operate the more than one treatment wands 40. In FIG. 1b, treatment wand 40a is coupled to generator unit 30 by cable 90a. Second and third treatment wands (not depicted) can be coupled to generator unit 30 by cables 90b and 90c, respectively. In operation, a first transducer 70 in first treatment wand 40a can be used to atomize a first material (e.g., a liquid), and a second transducer 70 can atomize another material, thereby allowing for dispensing of two different cellular or other solutions, or the same solutions with different ultrasound characteristics (e.g., differently sized transducers). In such an embodiment, an operator can use one wand 40 in each hand, sequentially use one then the other wand 40, or have multiple operators so that each wand is operated by its own operator. Multiple transducer embodiments can provide advantages, including being able to treat larger areas more quickly, allowing for dispensing different fluids and materials and treating with different ultrasound energies simultaneously, dispensing a first fluid without ultrasound and a second with ultrasound, among other advantages.

Some embodiments of system 10 have three modes of operation: a TREATMENT mode; an INFORMATION mode; and a TERMINAL mode. If the user enters the TREATMENT or normal operating mode upon power up, the user can select the length of time for a treatment and energize the acoustic output to treat a patient. In some embodiments, a user can select one or more additional parameters specific to a treatment to be provided, including parameters associated with a material to be delivered or otherwise used, including a cellular or biologic material. These parameters can include frequency, intensity, pulse length, beam characteristics, and application time on the skin. If the INFORMATION mode is entered on power up with a flash key plug to the USB port, user information can be downloaded that has been stored in the memory to flash or new software can be uploaded from the flash key to the system. Finally, a TERMINAL mode can be selected that is an engineering mode for internal device calibration, system characterization, and system evaluation.

System 10 may also save all information of the device hardware and software as well as the user's input and treatments during operation. In some embodiments, system 10 has enough memory storage for all information saved for at least one year of operation. For example, system 10 may implement 2MB bits EEPROM and flexible size memory in some embodiments.

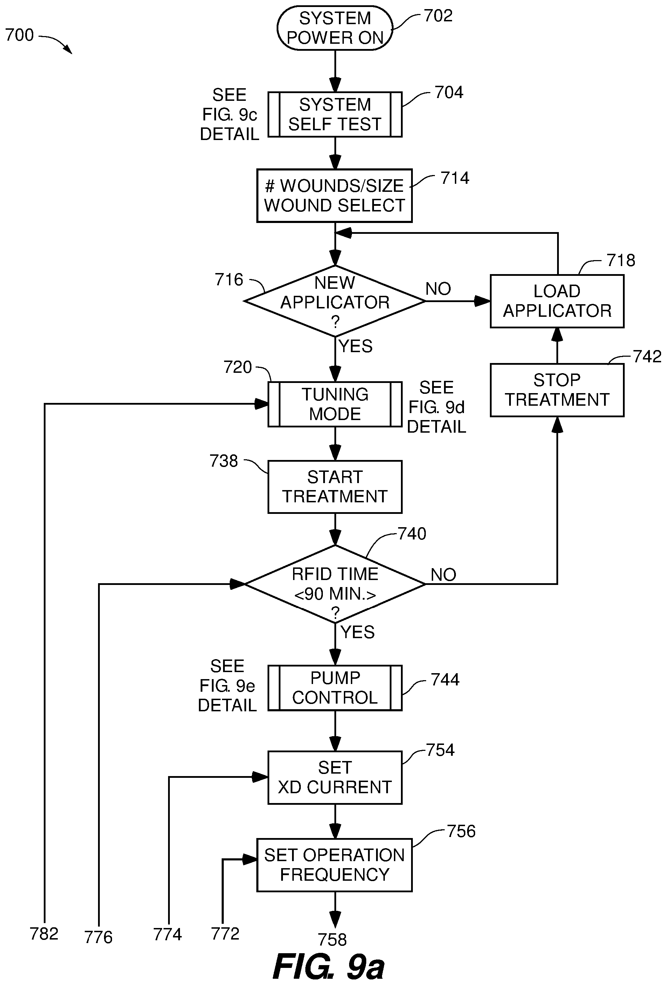

FIGS. 9a-g combine to provide a flow diagram operational method 700 of ultrasonic system 10. Operation begins by first powering on the system at 702, followed by conducting a system self-test at 704.

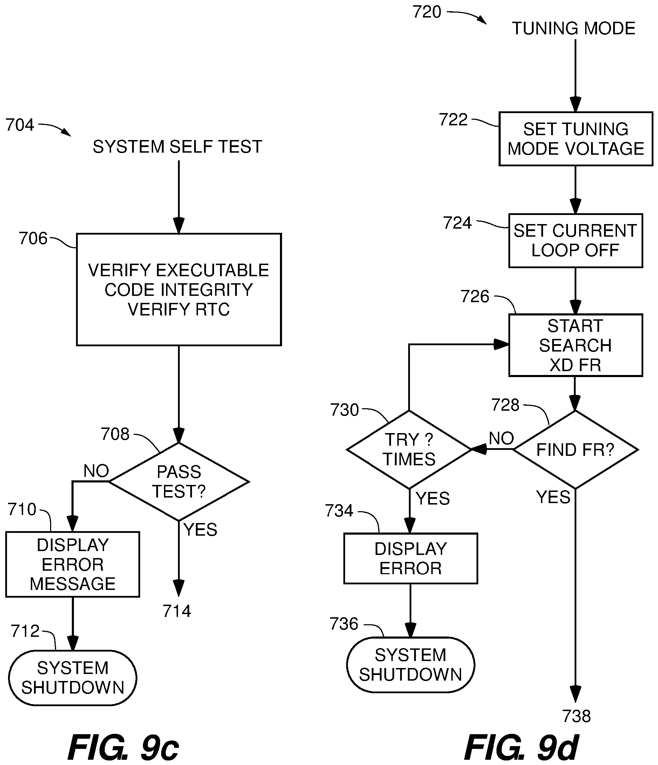

FIG. 9c shows the steps of self-test 704. First, system 10 can verify the integrity of the executable code and verifies RTC at 706. Next, at 708, if the self-test is passed, operation continues on to 714. If the self-test is not passed, an error message is displayed at 710 on display 206 and the system is shut down at 712.

If 714 is reached (in FIG. 9a), the number of wounds and size of wounds are input. If a new applicator 80 is present at 716, operation proceeds, if not, a new applicator 80 is loaded at 718. Next, at 720, tuning mode commences.

FIG. 9d shows tuning mode 720. First, the tuning mode voltage is set at 722. Next the current loop is set off at 724, followed by a search for the resonance frequency Fr of ultrasonic transducer 70 at 726. If the resonance frequency is found at 728, the system continues on to 738. If the resonance frequency is not found, the system will try again for a set number of times at 730. If resonance frequency is not found, after these attempts, an error is displayed on the system display 206 at 734, followed by system shutdown at 736.

If 720 is reached (in FIG. 9a), treatment is started following a successful tuning mode. Next, at 740, the system checks the RFID tag on the applicator 80 to ensure that the treatment has proceeded for less than ninety minutes. If not, treatment is stopped at 742 and a new applicator is loaded at 718 before reengaging the operation at 716. If the RFID tag indicates treatment of less than 90 minutes at 740, then operation continues on to pump control at 744.

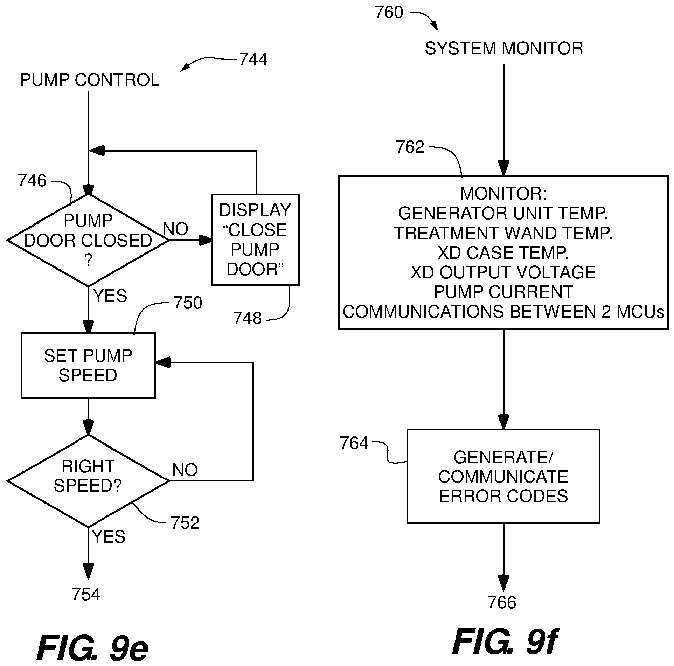

FIG. 9e shows the pump control 744. First, the system 10 checks that the pump doors of the peristaltic pumps 50 located on the exterior of the console / generator unit 50 are closed at 746. If not, the display 206 indicates a message to close the pump doors at 748. If the pump doors are closed, operation continues to 750 where the pump speed is set. The system then checks the pump speed at 752, and the pump speed is set again if necessary, before proceeding on to 754 when the pump controls is complete.

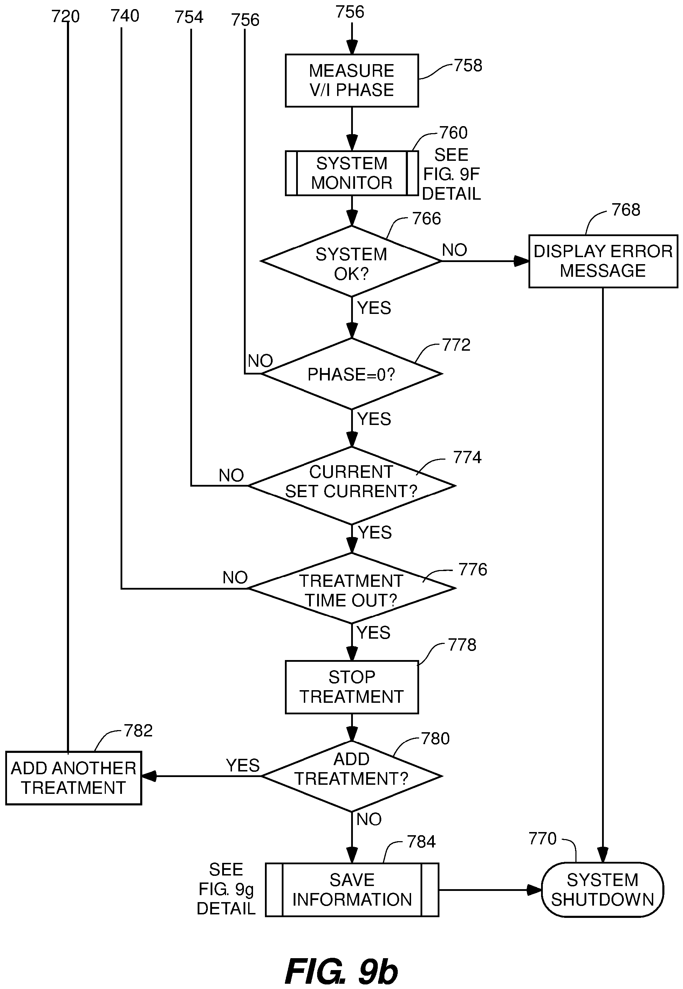

When 754 is reached (FIG. 9a), the current is set for the ultrasonic transducer 70. Next, the operation frequency is set at 756 and the voltage and current phase is measured at 758. See FIG. 9b. Next, monitoring the system commences at 760.

FIG. 9f shows monitoring the system at 760. First the system monitors: the temperature of the generator unit 30; the temperature of the treatment wand 40; the temperature of the case of the ultrasonic transducer 70; the output voltage of the ultrasonic transducer 70; the current of the pump 50; and the communication between the two microprocessors 200 and 316 (MCU2 and MCU1). As previously mentioned, additional sensing (e.g., tissue temperature) also can be monitored and controlled by the system in various embodiments. Next, error codes are generated and communicated at 764 before returning to 766.

When 766 is reached (FIG. 9b), if the system is not determined to be in order, an error message is communicated on the display 206 at 768 and the system is shut down at 770. If, however, the system is determined to be ok at 766, the system checks to ensure the voltage/current phase angle is 0° at 774. If not, operation reverts to 756 in which the operation frequency is adjusted to so that a voltage/current phase angle of 0° can be achieved. If voltage/current phase angle is set to 0° at 772, the system checks to ensure the current sensed is equivalent to the current that was set for the system at 774. If the current does not match, operation reverts to 754 and the transducer sets the current again before continuing. If the current is appropriate at 774, the system then tests to see if the treatment has timed out at 776. If it has not timed out, operation reverts to 740 and the test of 90 minute RFID time limit is conducted. If treatment has timed out at 776, the treatment is stopped at 778 followed by the option to add a further treatment at 780. If another treatment is desired, another treatment is added at 782 and operation reverts to the tuning mode at 720. If no further treatment is desired, information is saved at 784.

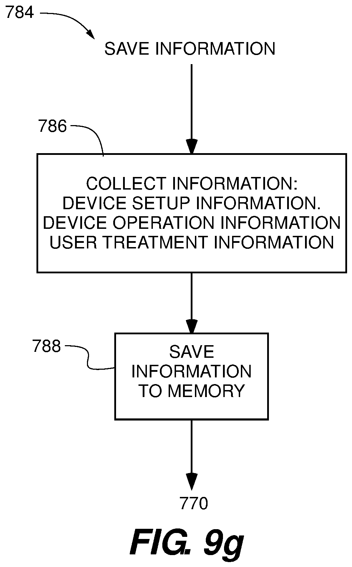

FIG. 9g shows saving information 784 in greater detail. First, the system collects device setup information, device operation information, and user treatment information at 786. Next, at 788, information is saved to EEPROM before continuing to system shutdown at 770.

As understood by the various system checks and protocols in this operational explanation, the operation of the system can be suspended at many points. Advantageously, in certain embodiments, both microprocessor 200 and microprocessor 316 are configured to individually suspend operation of the ultrasonic system in fault condition situations. This arrangement provides enhanced safety not present in other types of designs.

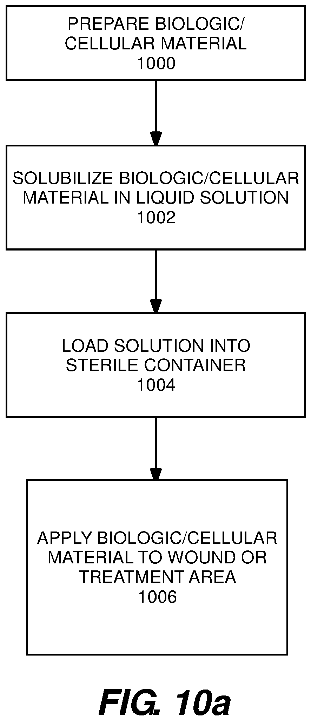

As previously discussed, embodiments relate to or include the application of and treatment with biologic/cellular material(s) and ultrasound therapies. At a high level, there can be three stages of therapy in some embodiments: (1) preparation, (2) treatment, and (3) post-treatment. FIGS. 10a-10d are flowcharts that illustrate examples related thereto. In all cases, what is depicted in the figures and discussed herein is but an example embodiment, and other embodiments may include additional tasks not specifically depicted or discussed, omit tasks that are depicted or discussed, or reorder tasks. Additionally, tasks or features from different figures may be combined in other embodiments.

As depicted in FIG. 10a, a biologic/cellular material is prepared at 1000. The material is solubilized into a liquid solution at 1002. In some embodiments the material can be solubilized in sterile saline solution, phosphate buffered saline, acidic solutions (e.g., having pH of greater than about 2.0), basic solutions (e.g., having a pH of less than about 13.0) or combinations thereof or other solutions. The solution can be loaded into a sterile container 130 at 1004. At 1006, the solution is applied to the wound or treatment area via e.g., system 10. In other embodiments, the solution or other biologic material can be applied to the wound or treatment area manually and then treated by system 10. For example, a biologic in, e.g., powder form can be manually applied, such as by sprinkling, to a treatment area and then treated by system 10.

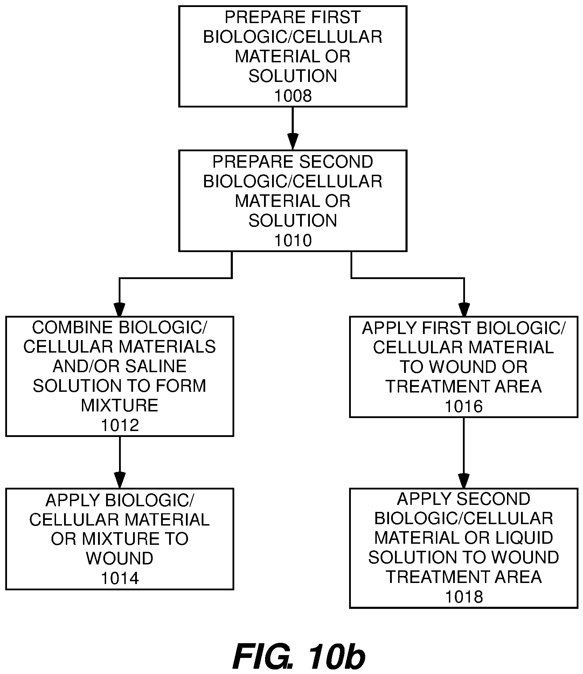

Another example depicted in FIG. 10b relates to embodiments in which a plurality of biologic/cellular materials is prepared and applied to a wound other area for treatment. At 1008, a first biologic/cellular material is prepared. At 1010, a second biologic/cellular material is prepared. In one embodiment, multiple biologics are combined or mixed at 1012. This can be accomplished, for example, by valving or other features of system 10, or the materials or solutions can be mixed and combined in a container or combined in some other way. At 1014, the mixture is applied to the wound or other treatment area by system 10.

In another embodiment, the first and second materials can be applied via system 10 sequentially, as shown at 1016 and 1018. For example, a liquid solution, such as saline or phosphate buffered saline, can be applied following biologic/cellular material application to the wound or treatment area. In other embodiments not depicted, a third or other additional materials can be applied, or different combinations of the tasks at 1012, 1014, 1016 and/or 1018 can be carried out in other embodiments. Delivery of the various materials and/or solutions can be accomplished and controlled by pumps, valving, multiple treatment wands and/or other features of various embodiments of system 10, as discussed above.

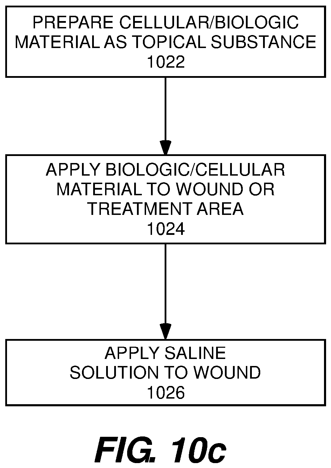

Another example embodiment is depicted in FIG. 10c. At 1022, a biologic/cellular material can be prepared as a topical application 1022. In some embodiments the topical application can be a cellular sheet, powderized/lyophilized material, jet milled, a cream, liquid solution, or any combination thereof, as previously discussed. After topical application of the biologic/cellular material at 1024, sterile saline solution, other liquid solutions, or other treatments or therapies can be applied to the wound or treatment area via system 10 at 1026.

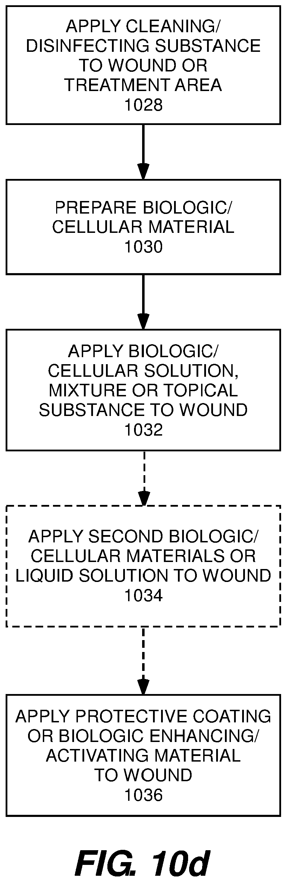

In the example embodiment of FIG. 10d, additional tasks can be included. For example, at 1028 a cleaning or disinfectant substance can be applied to the treatment area prior to application of the biologic/cellular material. A biologic/cellular material can then be prepared at 1030 and applied to the wound or treatment area at 1032. A liquid solution or second biologic/cellular material optionally can be applied at 1034 to the wound or treatment area. Following either task 1032 or task 1034, a protective coating or biologic enhancing or activating material can be applied to the wound or treatment area at 1036.

Thus, a variety of wounds, skin and other tissue conditions, and other issues can be treated by application of one or more biologic and/or cellular materials with an ultrasound system. These materials and the system can promote healing in a variety of ways, such as with faster healing, reduced pain, modulation of factors that affect healing processes (e.g., inflammation) and/or reduced likelihood of infection. For example, broad application of a biomaterial may reduce likelihood of adhesions when applied intraoperatively before closing a surgical site. This can minimize patient risk by being able to treat with material from one human donor rather than requiring more material and, hence, the potential for more than one donor (which multiplies the potential risk). In another example, penetration of some biologic and/or cellular materials can be enhanced (e.g., in the treatment of deep wounds or conditions) when administered by or with ultrasound. In yet another example, the systems, materials and methods disclosed herein can be used for debriding wounds or tissue areas, which also can promote wound healing. In a further example, embodiments can provide more effective ways to deliver topical treatment of defects associated with inflammatory conditions, which may allow drug therapy doses (and their side effects) to be decreased. In an even further example, embodiments can be used to deliver biologic drugs to bypass use of the gastrointestinal (GI) tract or injections (e.g., intravenous or intramuscular). Additionally, embodiments can reduce the aesthetic insult of scarring and provide more functional (regenerative) benefits than fibrotic results from an impaired healing process. Also, embodiments may be used to activate a bioglue or other molecule or material that can form an effective "new skin" barrier to a wound or tissue. These are only some examples, and many other examples have been given herein throughout. Still other examples and potential uses of the materials, systems, devices and/or methods discussed herein will be appreciated by those having skill in the art.

In an embodiment, a method of treating a skin, mucosa, or other condition comprises using an ultrasound delivery device to apply to an area of skin, mucosa or other tissue affected by the condition a mist that comprises a micronized cellular or biological material. The cellular or biological material can comprise a placental extracellular matrix composition or a placental connective tissue matrix composition. The placental extracellular matrix composition or placental connective tissue matrix composition can be prepared from whole placenta, placental deciduas, placental amniotic membrane, or placental chorionic membrane. The cellular or biological material can comprise a population of adherent cells. The cellular or biological material can comprise a mixture of adherent and non-adherent cells. The cellular or biological material can comprise platelet rich plasma or placental perfusate. The cellular or biological material can be micronized by grinding, milling, freeze drying, or heat drying. The skin condition can comprise a wound.

In an embodiment, a medical ultrasound device for delivering non-contact ultrasound therapies to a skin or other condition comprises at least one treatment wand comprising an ultrasonic transducer; at least one reservoir that contains a fluid or suspension comprising a micronized cellular or biological material; and a pump that is in fluid communication with the reservoir and the treatment wand to deliver the fluid to the treatment wand such that the ultrasonic transducer atomizes the cellular or biological material as the cellular or biological material passes through the treatment wand for delivery to the wound or other tissue. The the device can comprise a plurality of treatment wands and a plurality of reservoirs, wherein each treatment wand is in fluid communication with one of the reservoirs. The at least one of the plurality of reservoirs can contain a fluid for cleaning or disinfecting the area of skin or other tissue that is affected by the condition. The fluid can be for debriding a wound or tissue. The at least one of the reservoirs can contain a fluid for providing a protective or other coating on a wound or other tissue. The at least one reservoir can be sterile. The device can further comprise an applicator configured to be coupled to the treatment wand, wherein the applicator comprises a radio frequency identification (RFID) tag and the treatment wand comprises a RFID transceiver that is used to identify the RFID tag on the applicator to ensure that the applicator is limited to a single use. The device can further comprise a microprocessor configured to control operation of the device. The at least one treatment wand can comprise a plurality of tubes and the device comprises a plurality of reservoirs, each tube in fluid communication with a different one of the plurality of reservoirs, and wherein the microprocessor is configured to control a delivery pattern of fluids from the plurality of reservoirs. The delivery pattern can be sequential delivery of individual fluids or simultaneous delivery of at least two fluids. The medical ultrasound device can comprise one treatment wand and a plurality of reservoirs, wherein the device further comprises at least one valve configured to selectively couple at least one of the plurality of reservoirs to the treatment wand The at least one valve can comprise a static mixer. The device can comprise a microprocessor configured to control operation of the device and the at least one valve. The at least one valve can be manually controllable. The medical ultrasound device can further comprise a nozzle coupled to the treatment wand and configured to provide fluid from the at least one reservoir to the at least one treatment wand. The medical ultrasound device can further comprise a temperature control unit configured to heat or cool the fluid from the at least reservoir before the fluid is delivered to the treatment wand. The medical ultrasound device can further comprise a temperature control device configured to be coupled with the at least one reservoir to heat or cool the fluid contained therewithin. The treatment wand can comprise a motion processing unit and a user interface, wherein the user interface is configured to receive data from the motion processing unit and provide feedback to a user regarding positioning of the treatment wand during use of the device. The motion processing unit can be configured to adjust a characteristic of the device based on the received data related to positioning of the treatment wand during use of the device. The characteristic can be at least one of a flow rate of the fluid or a frequency of the ultrasonic transducer. The feedback can be at least one of visual feedback, audible feedback or haptic feedback. The at least one reservoir can comprise a machine-readable component, and the device can comprise a reader configured to read the machine-readable component and do at least one of: confirm that the fluid is for use with a particular patient; confirm a content of the reservoir; prompt a user to read, using the reader, a corresponding machine-readable patient identifier before use of the device; or receive device programming information from the machine-readable label. The machine-readable component can be a wirelessly machine-readable component, a radio frequency identification (RFID) tag, a bar code, or a Quick Response (QR) code. The medical ultrasound device can further comprise a reader configured to read a machine-readable component on a component or accessory of the device to confirm that the component or accessory is usable with the device. The medical ultrasound device can further comprise at least one mixer to mix the fluid in the at least one reservoir. The at least one mixer can mix the micronized cellular or biological material and the fluid. The at least one mixer can mix the fluid in a first one of the at least one reservoir with the fluid in at least a second one of the at least one reservoir. The at least one mixer can be a static mixer or a vibration mixer. At least one characteristic of the device can be variable to produce a variable spray pattern for delivery to the wound or other tissue. At least one characteristic of the device can be a size of an orifice of an applicator of the treatment wand, a shape of the orifice of the applicator of the treatment wand, a characteristic of the applicator of the treatment wand, a characteristic of a tip of an ultrasonic transducer of the treatment wand, or a flow rate of the fluid. The medical ultrasound device can further comprise a disposable kit comprising a single-use applicator coupleable to the treatment wand, tubing to convey the fluid from the reservoir to the treatment wand, and a spike to pierce the reservoir to couple the tubing to the reservoir. The kit can further comprise at least one of a valve or a disposable mixer.

In an embodiment, a method of promoting wound healing comprises providing a micronized cellular or biological material to be applied to the wound as a mist formed by an ultrasound delivery device. The cellular or biological material can comprise a placental extracellular matrix composition or a placental connective tissue matrix composition. The placental extracellular matrix composition or placental connective tissue matrix composition can be prepared from whole placenta, placental desidua, placental amniotic membrane, or placental chorionic membrane. The cellular or biological material can comprise a population of adherent cells. The cellular or biological material can comprise a mixture of adherent and non-adherent cells. The cellular or biological material can comprise platelet rich plasma or placental perfusate. The cellular or biological material can be micronized by grinding, milling, freeze drying, or heat drying.

In an embodiment, a method of promoting wound healing comprises providing a micronized cellular or biological material to be applied to the wound manually followed by a mist formed by an ultrasound delivery device. The cellular or biological material can comprise a placental extracellular matrix composition or a placental connective tissue matrix composition. The placental extracellular matrix composition or placental connective tissue matrix composition can be prepared from whole placenta, placental amniotic membrane, or placental chorionic membrane. The cellular or biological material can comprise a population of adherent cells. The cellular or biological material can comprise a mixture of adherent and non-adherent cells. The cellular or biological material can comprise platelet rich plasma or placental perfusate. The cellular or biological material can be micronized by grinding, jet milling, freeze drying, or heat drying.

In embodiments, system 10 and/or its components or systems include computing devices, microprocessors, modules and other computer or computing devices, which can be any programmable device that accepts digital data as input, is configured to process the input according to instructions or algorithms, and provides results as outputs. In an embodiment, computing and other such devices discussed herein can be, comprise, contain or be coupled to a central processing unit (CPU) configured to carry out the instructions of a computer program. Computing and other such devices discussed herein are therefore configured to perform basic arithmetical, logical, and input/output operations.

Computing and other devices discussed herein can include memory. Memory can comprise volatile or non-volatile memory as required by the coupled computing device or processor to not only provide space to execute the instructions or algorithms, but to provide the space to store the instructions themselves. In embodiments, volatile memory can include random access memory (RAM), dynamic random access memory (DRAM), or static random access memory (SRAM), for example. In embodiments, non-volatile memory can include read-only memory, flash memory, ferroelectric RAM, hard disk, floppy disk, magnetic tape, or optical disc storage, for example. The foregoing lists in no way limit the type of memory that can be used, as these embodiments are given only by way of example and are not intended to limit the scope of the invention.

In embodiments, the system or components thereof can comprise or include various modules or engines, each of which is constructed, programmed, configured, or otherwise adapted, to autonomously carry out a function or set of functions. The term "engine" as used herein is defined as a real-world device, component, or arrangement of components implemented using hardware, such as by an application specific integrated circuit (ASIC) or field-programmable gate array (FPGA), for example, or as a combination of hardware and software, such as by a microprocessor system and a set of program instructions that adapt the engine to implement the particular functionality, which (while being executed) transform the microprocessor system into a special-purpose device. An engine can also be implemented as a combination of the two, with certain functions facilitated by hardware alone, and other functions facilitated by a combination of hardware and software. In certain implementations, at least a portion, and in some cases, all, of an engine can be executed on the processor(s) of one or more computing platforms that are made up of hardware (e.g., one or more processors, data storage devices such as memory or drive storage, input/output facilities such as network interface devices, video devices, keyboard, mouse or touchscreen devices, etc.) that execute an operating system, system programs, and application programs, while also implementing the engine using multitasking, multithreading, distributed (e.g., cluster, peer-peer, cloud, etc.) processing where appropriate, or other such techniques. Accordingly, each engine can be realized in a variety of physically realizable configurations, and should generally not be limited to any particular implementation exemplified herein, unless such limitations are expressly called out. In addition, an engine can itself be composed of more than one sub-engines, each of which can be regarded as an engine in its own right. Moreover, in the embodiments described herein, each of the various engines corresponds to a defined autonomous functionality; however, it should be understood that in other contemplated embodiments, each functionality can be distributed to more than one engine. Likewise, in other contemplated embodiments, multiple defined functionalities may be implemented by a single engine that performs those multiple functions, possibly alongside other functions, or distributed differently among a set of engines than specifically illustrated in the examples herein.

Various embodiments of systems, devices, and methods have been described herein. These embodiments are given only by way of example and are not intended to limit the scope of the claimed invention.

## Claims

1. A medical ultrasound device (20) for delivering non-contact ultrasound therapies to a skin, mucosa, or other tissue affected by a wound, ulcer, or other condition, the device comprising:

   at least one treatment wand (40) comprising an ultrasonic transducer (70);
   at least one reservoir (130) that contains a fluid comprising a micronized cellular or biological material; and
   a pump (50) that is in fluid communication with the reservoir and the treatment wand to deliver the fluid to the treatment wand such that the ultrasonic transducer forms the fluid including the cellular or biological material into a mist as the fluid passes through the treatment wand for delivery to the skin, mucosa, or other tissue.

2. The medical ultrasound device of claim 1, wherein the device comprises a plurality of treatment wands (40) and a plurality of reservoirs (130), wherein each treatment wand is in fluid communication with one of the reservoirs.

3. The medical ultrasound device of claim 2, wherein at least one of the plurality of reservoirs (130) contains a fluid for cleaning or disinfecting the area of skin or other tissue that is affected by the condition, for debriding a wound or tissue, or for providing a protective or other coating on a wound or other tissue.

4. The medical ultrasound device of claim 1, wherein the at least one reservoir (130) is sterile.

5. The medical ultrasound device of claim 1, wherein the device further comprises an applicator (80) configured to be coupled to the treatment wand, wherein the applicator comprises a radio frequency identification (RFID) tag and the treatment wand (40) comprises a RFID transceiver that is used to identify the RFID tag on the applicator to ensure that the applicator is limited to a single use.

6. The medical ultrasound device of claim 1, wherein the device further comprises a microprocessor (200) configured to control operation of the device.

7. The medical ultrasound device of claim 6, wherein the at least one treatment wand (40) comprises a plurality of tubes (120) and the device comprises a plurality of reservoirs (130), each tube in fluid communication with a different one of the plurality of reservoirs, and wherein the microprocessor (200) is configured to control a delivery pattern of fluids from the plurality of reservoirs.

8. The medical ultrasound device of claim 1, comprising one treatment wand (40) and a plurality of reservoirs (130) , wherein the device further comprises at least one valve (102) configured to selectively couple at least one of the plurality of reservoirs to the treatment wand.

9. The medical ultrasound device of claim 8, wherein the at least one valve (102) comprises a static mixer (104).

10. The medical ultrasound device of claim 9, wherein the device comprises a microprocessor (200) configured to control operation of the device and the at least one valve (102).

11. The medical ultrasound device of claim 8, wherein the at least one valve (102) is manually controllable.

12. The medical ultrasound device of claim 1, further comprising a nozzle (80) coupled to the treatment wand (40) and configured to provide fluid from the at least one reservoir (130) to the at least one treatment wand.

13. The medical ultrasound device of claim 1, further comprising a temperature control unit (52) configured to heat or cool the fluid from the at least reservoir (130) before the fluid is delivered to the treatment wand (40).

14. The medical ultrasound device of claim 1, further comprising a temperature control device (53) configured to be coupled with the at least one reservoir (130) to heat or cool the fluid contained therewithin.

15. The medical ultrasound device of claim 1, wherein the treatment wand (40) comprises a motion processing unit (42) and a user interface (110), wherein the user interface is configured to receive data from the motion processing unit and provide feedback to a user regarding positioning of the treatment wand during use of the device, wherein the motion processing unit is configured to adjust a characteristic of the device based on the received data related to positioning of the treatment wand during use of the device, the characteristic being at least one of a flow rate of the fluid or a frequency of the ultrasonic transducer .

16. The medical ultrasound device of claim 15, wherein the feedback is at least one of visual feedback, audible feedback or haptic feedback.

17. The medical ultrasound device of claim 1, wherein the at least one reservoir (130) comprises a machine-readable component, and the device comprises a reader configured to read the machine-readable component and do at least one of:

   confirm that the fluid is for use with a particular patient;
   confirm a content of the reservoir;
   prompt a user to read, using the reader, a corresponding machine-readable patient identifier before use of the device; or
   receive device programming information from the machine-readable label.

18. The medical ultrasound device of claim 17, wherein the machine-readable component is a wirelessly machine-readable component, a radio frequency identification (RFID) tag, a bar code, or a Quick Response (QR) code.

19. The medical ultrasound device of claim 1, further comprising a reader configured to read a machine-readable component on a component or accessory of the device to confirm that the component or accessory is usable with the device.

20. The medical ultrasound device of claim 1, further comprising at least one mixer (104) to mix the fluid in the at least one reservoir (130), wherein the at least one mixer mixes the micronized cellular or biological material and the fluid.

21. The medical ultrasound device of claim 20, wherein the at least one mixer (104) mixes the fluid in a first one of the at least one reservoir (130) with the fluid in at least a second one of the at least one reservoir.

22. The medical ultrasound device of claim 20, wherein the at least one mixer (104) is a static mixer or a vibration mixer.

23. The medical ultrasound device of claim 1, wherein at least one characteristic of the device is variable to produce a variable spray pattern for delivery to the wound or other tissue, wherein the at least one characteristic is a size of an orifice of an applicator (80) of the treatment wand (40), a shape of the orifice of the applicator of the treatment wand, a characteristic of the applicator of the treatment wand, a characteristic of a tip of an ultrasonic transducer of the treatment wand, or a flow rate of the fluid.

24. The medical ultrasound device of claim 1, further comprising a disposable kit comprising a single-use applicator (80) coupleable to the treatment wand (40), tubing to convey the fluid from the reservoir to the treatment wand, and a spike to pierce the reservoir to couple the tubing to the reservoir.

25. The medical ultrasound device of claim 24, wherein the kit further comprises at least one of a valve or a disposable mixer.

**Patentansprüche**

1. Medizinische Ultraschallvorrichtung (20) zum Bereitstellen von kontaktlosen Ultraschalltherapien an einer Haut, Schleimhaut oder einem anderen Gewebe, das von einer Wunde, einem Geschwür oder einem anderen Leiden betroffen ist, die Vorrichtung Folgendes umfassend:

   mindestens einen Behandlungsstab (40), der einen Ultraschallwandler (70) umfasst;
   mindestens ein Reservoir (130), das ein Fluid enthält, das ein mikronisiertes Zell- oder biologisches Material umfasst; und
   eine Pumpe (50), die in Fluidverbindung mit dem Reservoir und dem Behandlungsstab ist, um dem Behandlungsstab das Fluid bereitzustellen, so dass der Ultraschallwandler das Fluid, welches das Zell- oder biologische Material umfasst, zu einem Nebel ausbildet, wenn das Fluid den Behandlungsstab zur Bereitstellung an die Haut, die Schleimhaut oder ein anderes Gewebe passiert.

2. Medizinische Ultraschallvorrichtung nach Anspruch 1, wobei die Vorrichtung mehrere Behandlungsstäbe (40) und mehrere Reservoire (130) umfasst, wobei jeder Behandlungsstab in Fluidverbindung mit einem der Reservoire ist.

3. Medizinische Ultraschallvorrichtung nach Anspruch 2, wobei mindestens eines der mehreren Reservoire (130) ein Fluid zum Reinigen oder Desinfizieren des Bereichs der Haut oder des anderen Gewebes, der von dem Leiden betroffen ist, zur Wund- oder Gewebeexzision oder zum Bereitstellen einer schützenden oder anderen Beschichtung auf einer Wunde oder einem anderen Gewebe enthält.

4. Medizinische Ultraschallvorrichtung nach Anspruch 1, wobei das mindestens eine Reservoir (130) steril ist.

5. Medizinische Ultraschallvorrichtung nach Anspruch 1, wobei die Vorrichtung weiterhin einen Applikator (80) umfasst, der eingerichtet ist, um mit dem Behandlungsstab gekoppelt zu sein, wobei der Applikator eine Hochfrequenzidentifikationsmarke (RFID-Marke) umfasst und der Behandlungsstab (40) einen RFID-Sendeempfänger umfasst, der verwendet wird, um die RFID-Marke auf dem Applikator zu identifizieren, um sicherzustellen, dass der Applikator zur einmaligen Verwendung beschränkt ist.

6. Medizinische Ultraschallvorrichtung nach Anspruch 1, wobei die Vorrichtung weiterhin einen Mikroprozessor (200) umfasst, der eingerichtet ist, um den Betrieb der Vorrichtung zu steuern.

7. Medizinische Ultraschallvorrichtung nach Anspruch 6, wobei der mindestens eine Behandlungsstab (40) mehrere Schläuche (120) umfasst und die Vorrichtung mehrere Reservoire (130) umfasst, wobei jeder Schlauch in Fluidverbindung mit einem anderen der mehreren Reservoire ist, und wobei der Mikroprozessor (200) eingerichtet ist, ein Bereitstellungsmuster von Fluiden aus den mehreren Reservoiren zu steuern.

8. Medizinische Ultraschallvorrichtung nach Anspruch 1, einen Behandlungsstab (40) und mehrere Reservoire (130) umfassend, wobei die Vorrichtung weiterhin mindestens ein Ventil (102) umfasst, das eingerichtet ist, um mindestens eines der mehreren Reservoire selektiv mit dem Behandlungsstab zu koppeln.

9. Medizinische Ultraschallvorrichtung nach Anspruch 8, wobei das mindestens eine Ventil (102) einen statischen Mischer (104) umfasst.

10. Medizinische Ultraschallvorrichtung nach Anspruch 9, wobei die Vorrichtung einen Mikroprozessor (200) umfasst, der eingerichtet ist, um den Betrieb der Vorrichtung und des mindestens einen Ventils (102) zu steuern.

EP 3 490 673 B1

11. Medizinische Ultraschallvorrichtung nach Anspruch 8, wobei das mindestens eine Ventil (102) manuell gesteuert werden kann.

12. Medizinische Ultraschallvorrichtung nach Anspruch 1, weiterhin eine Düse (80) umfassend, die mit dem Behandlungsstab (40) gekoppelt ist und eingerichtet ist, um dem mindestens einen Behandlungsstab Fluid aus dem mindestens einen Reservoir (130) bereitzustellen.

13. Medizinische Ultraschallvorrichtung nach Anspruch 1, weiterhin eine Temperaturregelungseinheit (52) umfassend, die eingerichtet ist, um das Fluid aus dem mindestens einen Reservoir (130) zu erwärmen oder zu kühlen, bevor das Fluid dem Behandlungsstab (40) bereitgestellt wird.

14. Medizinische Ultraschallvorrichtung nach Anspruch 1, weiterhin eine Temperaturregelungsvorrichtung (53) umfassend, die eingerichtet ist, um mit dem mindestens einen Reservoir (130) gekoppelt zu sein, um das darin enthaltene Fluid zu erwärmen oder zu kühlen.

15. Medizinische Ultraschallvorrichtung nach Anspruch 1, wobei der Behandlungsstab (40) eine Bewegungsverarbeitungseinheit (42) und eine Benutzerschnittstelle (110) umfasst, wobei die Benutzerschnittstelle eingerichtet ist, um Daten von der Bewegungsverarbeitungseinheit zu empfangen und einem Benutzer eine Rückmeldung hinsichtlich einer Positionierung des Behandlungsstabs bei einer Verwendung der Vorrichtung bereitzustellen, wobei die Bewegungsverarbeitungseinheit eingerichtet ist, um eine Eigenschaft der Vorrichtung auf der Grundlage der empfangenen Daten hinsichtlich einer Positionierung des Behandlungsstabs bei einer Verwendung der Vorrichtung einzustellen, wobei die Eigenschaft mindestens eine Flussrate des Fluids oder eine Frequenz des Ultraschallwandlers ist.

16. Medizinische Ultraschallvorrichtung nach Anspruch 15, wobei die Rückmeldung mindestens eine von einer visuellen Rückmeldung, einer akustischen Rückmeldung oder einer haptischen Rückmeldung ist.

17. Medizinische Ultraschallvorrichtung nach Anspruch 1, wobei das mindestens eine Reservoir (130) eine maschinenlesbare Komponente umfasst und die Vorrichtung ein Lesegerät umfasst, das eingerichtet ist, um die maschinenlesbare Komponente zu lesen und mindestens eines des Folgenden zu tun:

zu bestätigen, dass das Fluid zur Verwendung mit einem bestimmten Patienten vorgesehen ist;
einen Inhalt des Reservoirs zu bestätigen;
einen Benutzer aufzufordern, unter Verwendung des Lesegeräts eine entsprechende maschinenlesbare Patientenkennung vor Verwendung der Vorrichtung zu lesen; oder
Informationen zum Programmieren der Vorrichtung von dem maschinenlesbaren Etikett zu empfangen.

18. Medizinische Ultraschallvorrichtung nach Anspruch 17, wobei die maschinenlesbare Komponente eine drahtlos maschinenlesbare Komponente, eine Hochfrequenzidentifikationsmarke (RFID-Marke), ein Strichcode oder ein Quick-Response-Code (QR-Code) ist.

19. Medizinische Ultraschallvorrichtung nach Anspruch 1, weiterhin ein Lesegerät umfassend, das eingerichtet ist, um eine maschinenlesbare Komponente auf einer Komponente oder einem Zubehör der Vorrichtung zu lesen, um zu bestätigen, dass die Komponente oder das Zubehör mit der Vorrichtung verwendet werden kann.

20. Medizinische Ultraschallvorrichtung nach Anspruch 1, weiterhin mindestens einen Mischer (104) umfassend, um das Fluid in dem mindestens einen Reservoir (130) zu mischen, wobei der mindestens eine Mischer das mikronisierte Zell- oder biologische Material und das Fluid mischt.

21. Medizinische Ultraschallvorrichtung nach Anspruch 20, wobei der mindestens eine Mischer (104) das Fluid in einem ersten des mindestens einen Reservoirs (130) mit dem Fluid in mindestens einem zweiten des mindestens einen Reservoirs mischt.

22. Medizinische Ultraschallvorrichtung nach Anspruch 20, wobei der mindestens eine Mischer (104) ein statischer Mischer oder ein Vibrationsmischer ist.

23. Medizinische Ultraschallvorrichtung nach Anspruch 1, wobei mindestens eine Eigenschaft der Vorrichtung variabel ist, um ein variables Sprühmuster zur Bereitstellung an die Wunde oder ein anderes Gewebe zu erzeugen, wobei die mindestens eine Eigenschaft eine Größe einer Öffnung eines Applikators (80) des Behandlungsstabs (40), eine Gestalt der Öffnung des Applikators des Behandlungsstabs, eine Eigenschaft des Applikators des Behandlungsstabs, eine Eigenschaft einer Spitze eines Ultraschallwandlers des Behandlungsstabs oder eine Flussrate des Fluids ist.

24. Medizinische Ultraschallvorrichtung nach Anspruch 1, weiterhin einen Einwegsatz umfassend, der einen Einwegapplikator (80), der mit dem Behandlungsstab (40) gekoppelt werden kann, einen Schlauch, um das Fluid aus dem Reservoir zu dem Behandlungsstab zu befördern, und einen Dorn umfasst, um das Reservoir zu durchstechen, um den Schlauch mit dem Reservoir zu koppeln.

25. Medizinische Ultraschallvorrichtung nach Anspruch 24, wobei der Satz weiterhin mindestens ein Ventil oder einen Einwegmischer umfasst.

**Revendications**

1. Dispositif médical à ultrasons (20) permettant d'administrer des thérapies à ultrasons sans contact à une peau, une muqueuse ou un autre tissu affecté par une plaie, un ulcère ou une autre affection, le dispositif comprenant :

   au moins une tige de traitement (40) comprenant un transducteur ultrasonore (70) ;
   au moins un réservoir (130) qui contient un fluide comprenant un matériau cellulaire ou biologique micronisé ; et
   une pompe (50) en communication fluidique avec le réservoir et la tige de traitement pour acheminer le fluide vers la tige de traitement de sorte que le transducteur ultrasonore forme un brouillard avec le fluide comprenant le matériau cellulaire ou biologique lorsque le fluide traverse la tige de traitement pour être administré à la peau, aux muqueuses ou à d'autres tissus.

2. Dispositif médical à ultrasons selon la revendication 1, le dispositif comprenant une pluralité de tiges de traitement (40) et une pluralité de réservoirs (130), chaque tige de traitement étant en communication fluidique avec l'un des réservoirs.

3. Dispositif médical à ultrasons selon la revendication 2, au moins un de la pluralité de réservoirs (130) contenant un fluide pour nettoyer ou désinfecter la zone de peau ou d'autres tissus qui est affectée, pour débrider une plaie ou un tissu, ou pour fournir un revêtement protecteur ou autre sur une plaie ou d'autres tissus.

4. Dispositif médical à ultrasons selon la revendication 1, l'au moins un réservoir (130) étant stérile.

5. Dispositif médical à ultrasons selon la revendication 1, le dispositif comprenant en outre un applicateur (80) configuré pour être couplé à la tige de traitement, l'applicateur comprenant une étiquette d'identification par radiofréquence (RFID) et la tige de traitement (40) comprenant un émetteur-récepteur RFID qui est utilisé pour identifier l'étiquette RFID sur l'applicateur afin de s'assurer que l'applicateur est limité à une seule utilisation.

6. Dispositif médical à ultrasons selon la revendication 1, le dispositif comprenant en outre un microprocesseur (200) configuré pour commander le fonctionnement du dispositif.

7. Dispositif médical à ultrasons selon la revendication 6, l'au moins une tige de traitement (40) comprenant une pluralité de tubes (120) et le dispositif comprenant une pluralité de réservoirs (130), chaque tube étant en communication fluidique avec un réservoir différent de la pluralité de réservoirs, et le microprocesseur (200) étant configuré pour commander un modèle de distribution de fluides à partir de la pluralité de réservoirs.

8. Dispositif médical à ultrasons selon la revendication 1, comprenant une tige de traitement (40) et une pluralité de réservoirs (130), le dispositif comprenant en outre au moins une valve (102) configurée pour coupler sélectivement au moins un de la pluralité de réservoirs à la tige de traitement.

9. Dispositif médical à ultrasons selon la revendication 8, l'au moins une valve (102) comprenant un mélangeur statique (104).

10. Dispositif médical à ultrasons selon la revendication 9, le dispositif comprenant un microprocesseur (200) configuré pour commander le fonctionnement du dispositif et de l'au moins une valve (102).

**11.** Dispositif médical à ultrasons selon la revendication 8, l'au moins une valve (102) étant commandable manuellement.

**12.** Dispositif médical à ultrasons selon la revendication 1, comprenant en outre une buse (80) couplée à la tige de traitement (40) et configurée pour fournir un fluide à partir de l'au moins un réservoir (130) à l'au moins une tige de traitement.

**13.** Dispositif médical à ultrasons selon la revendication 1, comprenant en outre une unité de commande de la température (52) configurée pour chauffer ou refroidir le fluide provenant de l'au moins un réservoir (130) avant que le fluide ne soit délivré à la tige de traitement (40).

**14.** Dispositif médical à ultrasons selon la revendication 1, comprenant en outre un dispositif de commande de la température (53) configuré pour être couplé avec l'au moins un réservoir (130) afin de chauffer ou de refroidir le fluide qu'il contient.

**15.** Dispositif médical à ultrasons selon la revendication 1, la tige de traitement (40) comprenant une unité de traitement des mouvements (42) et une interface utilisateur (110), l'interface utilisateur étant configurée pour recevoir des données de l'unité de traitement des mouvements et fournir un retour d'information à un utilisateur concernant le positionnement de la tige de traitement pendant l'utilisation du dispositif, l'unité de traitement des mouvements étant configurée pour ajuster une caractéristique du dispositif sur la base des données reçues relatives au positionnement de la tige de traitement pendant l'utilisation du dispositif, la caractéristique étant au moins l'une d'un débit du fluide ou d'une fréquence du transducteur ultrasonore.

**16.** Dispositif médical à ultrasons selon la revendication 15, le retour d'information étant au moins l'un d'un retour d'information visuel, d'un retour d'information sonore ou d'un retour d'information haptique.

**17.** Dispositif médical à ultrasons selon la revendication 1, l'au moins un réservoir (130) comprenant un composant lisible par machine, et le dispositif comprenant un lecteur configuré pour lire le composant lisible par machine et faire au moins l'une des choses suivantes :

confirmer que le fluide est destiné à être utilisé avec un patient particulier ;
confirmer le contenu du réservoir ;
inviter un utilisateur à lire, à l'aide du lecteur, un identifiant de patient lisible par machine correspondant avant d'utiliser le dispositif ; ou
recevoir des informations de programmation du dispositif à partir de l'étiquette lisible par machine.

**18.** Dispositif médical à ultrasons selon la revendication 17, le composant lisible par machine étant un composant lisible par machine sans fil, une étiquette d'identification par radiofréquence (RFID), un code-barres ou un code de réponse rapide (QR).

**19.** Dispositif médical à ultrasons selon la revendication 1, comprenant en outre un lecteur configuré pour lire un composant lisible par machine sur un composant ou un accessoire du dispositif pour confirmer que le composant ou l'accessoire est utilisable avec le dispositif.

**20.** Dispositif médical à ultrasons selon la revendication 1, comprenant en outre au moins un mélangeur (104) pour mélanger le fluide dans l'au moins un réservoir (130), l'au moins un mélangeur mélangeant le matériau cellulaire ou biologique micronisé et le fluide.

**21.** Dispositif médical à ultrasons selon la revendication 20, l'au moins un mélangeur (104) mélangeant le fluide dans un premier de l'au moins un réservoir (130) avec le fluide dans au moins un second de l'au moins un réservoir.

**22.** Dispositif médical à ultrasons selon la revendication 20, l'au moins un mélangeur (104) étant un mélangeur statique ou un mélangeur à vibrations.

**23.** Dispositif médical à ultrasons selon la revendication 1, au moins une caractéristique du dispositif étant variable pour produire un modèle de pulvérisation variable pour la livraison à la plaie ou à taille d'un orifice d'un applicateur (80) de la tige de traitement (40), une forme de l'orifice de l'applicateur de la tige de traitement, une caractéristique de l'applicateur de la tige de traitement, une caractéristique d'un embout d'un transducteur ultrasonore de la tige de traitement, ou un débit du fluide.

**24.** Dispositif médical à ultrasons selon la revendication 1, comprenant en outre un kit jetable comprenant un applicateur à usage unique (80) pouvant être couplé à la tige de traitement (40), une tubulure pour acheminer le fluide du réservoir à la tige de traitement, et une pointe pour percer le réservoir afin de coupler la tubulure au réservoir.

**25.** Dispositif médical à ultrasons selon la revendication 24, le kit comprenant en outre au moins l'un des éléments suivants : une valve ou un mélangeur jetable.

**FIG. 1a**

**FIG. 1b**

**FIG. 1c**

**FIG. 1d-1**

**FIG. 1d-2**

**FIG. 1d-3**

**FIG. 1d-4**

**FIG. 1d-5**

83

80

120a

120c

120b

**FIG. 1e-1**

80    83

82

**FIG. 1e-2**

120c

120b    120a

**FIG. 1e-3**

140

82

120

**FIG. 1e-4**

**FIG. 1f-1**

**FIG. 1f-2**

**FIG. 1f-3**

**FIG. 1f-4**

FIG. 1g

**FIG. 2**

10 ⌐

130 ⌐

STERILE
CONTAINER(S)
W/ MATERIAL
FOR DELIVERY

SENSORS
AND/OR
HEATING/
COOLING
ELEMENTS

80 ⌐

APPLICATOR
(WITH RFID
TAG)

TUBE(S)

120 ⌐

50 ⌐

PUMP(S)/
MIXER(S)/
VALVING

ULTRASONIC
TRANSDUCER

70 ⌐

140

100

120VAC/
240VAC
50/60Hz

GENERATOR
UNIT

TREATMENT
WAND UNIT

USB(A) SERIAL
PORT

30

CABLE

90

40

110 ⌐

USER
INTERFACE

**FIG. 3**

FIG. 4

316

MICRO-
CONTROLLER

FREQUENCY SET

370

AMPLITUDE SET

372

318

DDS

F OUT

374

**FIG. 5**

**FIG. 6**

FIG. 7

FIG. 8

**FIG. 9a**

**FIG. 9b**

720 — TUNING MODE

722 — SET TUNING
MODE VOLTAGE

724 — SET CURRENT
LOOP OFF

726 — START
SEARCH
XD FR

730 — TRY ?
TIMES

728 — FIND FR?

NO

YES

734 — DISPLAY
ERROR

736 — SYSTEM
SHUTDOWN

YES

738

**FIG. 9d**

704 — SYSTEM SELF TEST

706 — VERIFY EXECUTABLE
CODE INTEGRITY
VERIFY RTC

708 — PASS
TEST?

NO

YES

710 — DISPLAY
ERROR
MESSAGE

714

712 — SYSTEM
SHUTDOWN

**FIG. 9c**

744

PUMP CONTROL

746

PUMP DOOR CLOSED ?

NO → DISPLAY "CLOSE PUMP DOOR"

748

YES

750

SET PUMP SPEED

RIGHT SPEED?

NO

752

YES

754

**FIG. 9e**

760

SYSTEM MONITOR

762

MONITOR:
GENERATOR UNIT TEMP.
TREATMENT WAND TEMP.
XD CASE TEMP.
XD OUTPUT VOLTAGE
PUMP CURRENT
COMMUNICATIONS BETWEEN 2 MCUs

764

GENERATE/ COMMUNICATE ERROR CODES

766

**FIG. 9f**

784 ⟶

SAVE INFORMATION

786 ⟍

COLLECT INFORMATION:
DEVICE SETUP INFORMATION.
DEVICE OPERATION INFORMATION
USER TREATMENT INFORMATION

788 ⟍

SAVE
INFORMATION
TO MEMORY

770

# FIG. 9g

PREPARE BIOLOGIC/
CELLULAR MATERIAL
1000

SOLUBILIZE BIOLOGIC/CELLULAR
MATERIAL IN LIQUID SOLUTION
1002

LOAD SOLUTION INTO
STERILE CONTAINER
1004

APPLY BIOLOGIC/CELLULAR
MATERIAL TO WOUND OR
TREATMENT AREA
1006

*FIG. 10a*

```
              ┌─────────────────────┐
              │   PREPARE FIRST     │
              │ BIOLOGIC/CELLULAR   │
              │   MATERIAL OR       │
              │    SOLUTION         │
              │      1008           │
              └─────────┬───────────┘
                        │
                        ▼
              ┌─────────────────────┐
              │  PREPARE SECOND     │
              │ BIOLOGIC/CELLULAR   │
              │   MATERIAL OR       │
              │    SOLUTION         │
              │      1010           │
              └──┬──────────────┬───┘
                 │              │
                 ▼              ▼
    ┌──────────────────┐  ┌──────────────────┐
    │ COMBINE BIOLOGIC/│  │APPLY FIRST BIOLOGIC/│
    │ CELLULAR MATERIALS│ │ CELLULAR MATERIAL│
    │  AND/OR SALINE   │  │   TO WOUND OR    │
    │ SOLUTION TO FORM │  │ TREATMENT AREA   │
    │    MIXTURE       │  │      1016        │
    │      1012        │  └────────┬─────────┘
    └────────┬─────────┘           │
             │                     ▼
             ▼            ┌──────────────────┐
    ┌──────────────────┐  │  APPLY SECOND    │
    │ APPLY BIOLOGIC/  │  │ BIOLOGIC/CELLULAR│
    │ CELLULAR MATERIAL│  │ MATERIAL OR LIQUID│
    │  OR MIXTURE TO   │  │ SOLUTION TO WOUND│
    │    WOUND         │  │ TREATMENT AREA   │
    │      1014        │  │      1018        │
    └──────────────────┘  └──────────────────┘
```

## *FIG. 10b*

FIG. 10c

APPLY CLEANING/
DISINFECTING SUBSTANCE
TO WOUND OR
TREATMENT AREA
1028

↓

PREPARE BIOLOGIC/
CELLULAR MATERIAL
1030

↓

APPLY BIOLOGIC/
CELLULAR SOLUTION,
MIXTURE OR TOPICAL
SUBSTANCE TO WOUND
1032

↓

APPLY SECOND BIOLOGIC/
CELLULAR MATERIALS OR
LIQUID SOLUTION TO WOUND
1034

↓

APPLY PROTECTIVE COATING
OR BIOLOGIC ENHANCING/
ACTIVATING MATERIAL
TO WOUND
1036

*FIG. 10d*

**EP 3 490 673 B1**

## REFERENCES CITED IN THE DESCRIPTION

**Patent documents cited in the description**

- US 6569099 B
- US 2015148712 A
- US 2007073197 A
- US 2014335046 A
- US 2009043248 A
- US 2012046601 A